# EUROPEAN PATENT APPLICATION

(11) **EP 3 323 424 A1**
(43) Date of publication of application: **23.05.2018**
(21) Application number: 16002451.9
(22) Date of filing: 17.11.2016
(51) Int. Cl.: A61K 39/00, A61K 38/16, A61K 39/008, A61K 39/02, A61K 39/39

(54) **CYAA POLYPEPTIDES AS IMMUNE ENHANCER**

(71) Applicant: Cyanimal IP, 31280 Dremil-Lafage (FR)
(72) Inventor: NALIN, Renaud, 31280 Dremil Lafage (FR); BOULLIER, Séverine, 31100 Toulouse (FR); CORNILLE, Patrick, 13760 Saint Cannat (FR)
(74) Representative: Barbot, Willy

(57) **Abstract**

The present invention relates to an immunogenic composition comprising i) a polypeptide comprising a sequence derived from ant adenylate cyclase toxin-hemolysin (CyaA) protein or a fragment thereof, and ii) at least one active agent and their use as a medicament like a vaccine.

## Description

### FIELD OF THE INVENTION

The present invention relates to vaccination and, in particular, to formulating vaccines so as to achieve an enhanced immunogenic response to an antigen.

### BACKGROUND

In the conventional method of protection against disease by vaccination, the central principle involves the injection or ingestion of a material that will elicit antibodies against said material, such that, when challenged later with a pathogenic organism containing homologous material, the individual to whom the material has been administered is protected against the pathogenic disease. The materials injected or ingested that have this property of eliciting antibodies are called antigens.

The first step in making a vaccine is to separate the disease-making, from the immune-inducing activity. In practice this means isolating or creating an organism, or part of one, that is unable to cause full-blown disease, but that still retains the antigens responsible for inducing the host's immune response. We distinguish two major groups of vaccines: whole organism vaccines and sub-unit vaccines. Whole organism vaccines are produced by killing/inactivating or attenuating/weakening organisms and Sub-unit vaccines include vaccines based on for example protein antigens and carbohydrate antigens. Some vaccines elicit a weak response meaning that materials apparently have little or no immunogenicity made to make high titers of antibody in in *vivo* systems.

Attempts have been made to enhance the immune response of weakly-immunogenic materials by the addition of so-called adjuvants. Some of these adjuvants are, however, highly toxic and can cause undesirable side effects or lesions. Aluminum hydroxide or aluminum phosphate are routinely used as adjuvants for human vaccines, but have been known to cause lumps at the site of injection. Freund's complete adjuvant (FCA), a mixture of a light petroleum oil and killed *Mycobacterium tuberculosis,* can often produce excellent titers for materials which do not normally give antibodies *in vivo* under any other conditions. Unfortunately lesions will often develop at the site of injection when using this material, making the procedure unacceptable for a safety use.

There are a number of other materials which have been investigated for use as adjuvants. In published European Patent Application No. 0149581, the use of muramyl di-peptide is disclosed. In published United Kingdom Patent Application No. 2,053,231, the use of a synthetic adjuvant consisting of tetrapeptides or pentapeptides is disclosed. In earlier patents, the use of oil emulsions is disclosed as having an adjuvanting effect. Unfortunately, it appears that the better a material is at behaving as an adjuvant to antigen immunogenicity, the worse are the side effects. For example, it has been shown that muramyl dipeptide is an excellent adjuvant but appears to have a number of undesirable properties which prevent its use in human vaccines.

In general, all the adjuvants presently in use in mammals fall into two distinct types. The first type involves the so-called "depot" effect and the second type depends on general immunological stimulation of the system under study. The adjuvants which rely on the depot effect are believed to bring the immune cells to the antigen site, where the depot effect relies on the injected antigen being trapped or insolubilized in a medium, giving sustained circulating levels. The second type of adjuvant, involving general stimulation of the immune system, appears to rely on an inflammatory reaction resulting in a series of cells being stimulated, such that any antigen has an improved chance of eliciting antibodies.

Efforts have been made to identify new immune modulators to avoid adjuvants for vaccines and immunotherapies that would overcome the drawbacks and deficiencies of these adjuvants.
Some of these strongly-immunogenic materials so-called Immune Response Enhancers (IRE) are mainly polysaccharide such as for example conjugate of the capsular polysaccharide of *Haemophilus influenzae* type b to diphtheria toxoid as described in US4,496,538 and US4,619,828, or a beta-1,4-linked D-mannuronic acid as described in WO06034395. Sometimes IREs are small molecule immune potentiators such as tryptanthrin as described in US 8,193,185 or palmitoyl-Cys(2[R],3-dilauroyloxy-propyl)-Abu-D-Glu-NH2 as described in WO2012129483 or imidizoquinolines as described by Hemmi H et al. ("small antiviral compounds activate immune cells via TLR7 MyD88 dependent signaling pathway", Nature Immunol. , 2002, 3: 196-200). It is important to note that the first generation of imidizoquinoline is licensed as an antiviral as well as anticancer topical therapy (Aldara) and not a vaccine potentiator.

Therefore, there is still a need for improve immune Response Enhancers (IRE) to develop formulation that elicits potent cell-mediated and humoral immune responses to a wide range of antigens in humans and domestic animals, but lacking the side effects of conventional adjuvants and other immune modulators.

### SUMMARY OF INVENTION

The invention relates to an immunogenic composition comprising i) a polypeptide comprising a sequence derived from an adenylate cyclase toxin (CyaA) protein or a fragment thereof, or a polynucleotide encoding for such a polypeptide and ii) at least one active agent.

The invention also relates to a method for vaccinating or treating a human and/or a non-human animal comprising administering an immunogenic composition as defined above.

The invention also relates to an immunogenic composition as defined above for the use of treating or vaccinating a human and/or a non-human animal against various diseases.

The invention may be used in human(s) and/or non-human animal(s) to treat or vaccinate against various diseases.

### DETAILLED DESCRIPTION

In a first aspect, the present invention provides an immunogenic composition comprising i) a polypeptide comprising a sequence derived from an adenylate cyclase toxin (CyaA) protein or a fragment thereof, or a polynucleotide encoding for such a polypeptide and ii) at least one active agent.

In a first preferred embodiment, the sequence derived from an adenylate cyclase toxin (CyaA) protein comprises a deletion in the N amino-terminal adenylate cyclase (AC) domain.

*Bordetella pertussis,* the etiological agent of the highly contagious respiratory disease known as pertussis or whooping cough, secretes an adenylate cyclase toxin (CyaA also called ACT or AC-Hly), which belongs to the broad and important RTX (Repeats in ToXin) family of toxins secreted by Gram-negative pathogens. CyaA primarily targets host myeloid cells expressing complement receptor 3 (CR3), also known as the αMβ2 integrin, CD11b/CD18, or Mac-1. Besides CR3-expressing cells, the toxin can also bind and intoxicate with cAMP, at reduced but detectable levels, a broad variety of other cells, including mammalian erythrocytes on which CyaA can exert its hemolytic activity.

The CyaA of Bordetella species in particular of Bordetella pertussis, is a 1706 residue-long (177-kDa, Accession number CPI05197, SEQ ID NO:1) and has been described as an amino acid sequence and a nucleotide sequence by Glaser P. et al., 1988, Molecular Microbiology 2(1), 19-30. Accordingly, when amino acid residues or sequences or nucleotides or nucleotide sequences of the CyaA protein of B. pertussis, are cited in the present invention their positions are given with respect to the sequences disclosed in said publication of Glaser P. et al., 1988.

As used herein, "CyaA protein" or "wild-type CyaA protein" refers to the adenylate cyclase toxin protein from a *Bordetella sp.,* such as CyaA protein from *Bordetella pertussis* (Accession number CPI 05197, SEQ ID NO:1), from *Bordetella bronchiseptica* (Accession number KDS81064, SEQ ID NO:2), from *Bordetella hinzii* (Accession number AAY57201, SEQ ID NO:1), or *Bordetella parapertussis* (Accession number CAB76450, SEQ ID NO:3).

CyaA is a bifunctional protein that consists of an amino-terminal adenylate cyclase (AC) domain of about 400 residues (N-terminal residues 1 to 373) and of a RTX hemolysin domain (Hly). Intriguingly, insertion of large passenger peptides removes the enzymatic activity but not the cell-invasive capacity of the AC domain. This has repeatedly been exploited for delivery of heterologous antigens into the cytosolic pathway of CD11b-expressing dendritic cells by CyaA/AC(-) toxoids, thus enabling their processing and presentation on major histocompatibility complex (MHC) class I molecules to cytotoxic CD8(+) T lymphocytes (CTLs).

As used herein, the expression "AC domain" refers to the amino-terminal adenylate cyclase domain of CyaA protein. The skilled person can simply identify such AC domain in the CyaA protein sequence. As an example, such AC domain corresponds to the amino acid 1 to 373 from SEQ ID NO:1, SEQ ID NO:2 or SEQ ID NO:3.

As used herein, the expression "a deletion in the AC domain", refers to a deletion inactivating the amino-terminal adenylate cyclase activity of the AC domain.

In a specific embodiment, said deletion in the AC domain, refers to a deletion of at least 100 amino acids, preferably of at least 200 amino acids, and most preferably of at least 300 amino acids in said domain -.e.g. SEQ ID NO:4-. More preferably, the polypeptide of the invention does not comprise the sequence of the AC domain -e.g. the sequence SEQ ID NO:4.

In a more particular embodiment, the sequence derived from an adenylate cyclase toxin (CyaA) protein consists of residues 235 to 1706 of SEQ ID NO:1, SEQ ID NO:2 or SEQ ID NO:3, more preferably residues 321 to 1706 of SEQ ID NO:1, SEQ ID NO:2 or SEQ ID NO:3, even more preferably residues 372 to 1706 of SEQ ID NO:1, SEQ ID NO:2 or SEQ ID NO:3 or 374 to 1706 of SEQ ID NO:1, SEQ ID NO:2 or SEQ ID NO:3.

A set of toxoids with overlapping deletions within the first 371 residues of CyaA have been constructed and showed that the structure of the AC domain does not contain any sequences indispensable for its translocation across target cell membrane. Moreover, replacement of the AC domain (residues 1 to 373- eg SEQ ID NO:4.) with heterologous polypeptides of 40, 146 or 203 residues yielded CyaA-ΔAC constructs that delivered passenger CTL epitopes into antigen-presenting cells (APCs) and induced strong antigen-specific CD8(+) CTL responses in vivo in mice and ex vivo in human peripheral blood mononuclear cell cultures (Holubova J. et al., 2012, Infect. Immun., 80:1181-92 - Jelinek J. et al., 2012, Bone Marrow Transpl., 47:243-50).

This shows that the RTX hemolysin (Hly) domain, which follows the AC domain, and consisting of residues 374 to 1706 of CyaA (SEQ ID NO:1, SEQ ID NO:2 or SEQ ID NO:3), harbors all structural information involved in translocation of the N-terminal AC domain across target cell membranes. These results decipher the extraordinary capacity of the AC domain of CyaA to transport large heterologous cargo polypeptides into the cytosol of CD11b(+) target cells and pave the way for the construction of CyaA-ΔAC based polyvalent immunotherapeutic T cell vaccines.

The Hly domain can form small cation selective membrane pores that allow efflux of potassium ions from cells and can cause colloid-osmotic cell lysis, such as hemolysis of erythrocytes. The capacity of CyaA to penetrate cellular membranes, to form pores, and to deliver the AC domain into the cytosol of target cells depends on covalent posttranslational fatty acylation of pro-CyaA at the ε-amino groups of the internal lysine residues 983 and 860 by a co-expressed protein toxin acyltransferase, CyaC. Toxin activities further require loading of calcium ions into the numerous binding sites formed by the glycine and aspartate rich nonapeptide repeats of the RTX domain.

Compared to other RTX toxins, CyaA is indeed a less potent hemolysin, forming smaller pores than all the other RTX cytolysins that do not have homologous segments at the N-terminal ends of their pore-forming domains. Indeed, CyaA forms cation-selective membrane pores of an inner diameter of 0.6 to 0.8 nm while the best characterized RTX hemolysins, such as E. coli HlyA or *Actinobacillus pleuropneumoniae* ApxIA, form larger pores with estimated inner diameters of 1.5 to 2 nm and about an order of magnitude higher unit conductance than CyaA (Benz R. et al., 1994, J. Biol. Chem., 269:27231-27239 - Masin J. et al., 2013,. Infect. Immun., 81: 4571-4582). When the N-terminal truncation was extended up to residue 489 of CyaA, the resulting CyaA-ΔN489 construct exhibited at least an order of magnitude higher membrane-permeabilizing capacity, forming larger pores and at a higher propensity than intact CyaA (Gray M. C. et al., 2001, J. Bacteriol., 183:5904-5910). It was observed that the translocation of the AC domain across the membrane was abolished by the deletion of residues 375 to 485 of CyaA (Karst J. C. et al., 2012, J Biol Chem., 287: 9200-9212 - Subrini, O. et al., 2013, J. Biol. Chem.,10: 1074).

As used herein, the expression "Hly linking segment" refers to the about 100 amino acid sequence following the AC domain in the CyaA protein, preferably refers to the residues 374 to 500 of SEQ ID NO:1, SEQ ID NO:2 or SEQ ID NO:3 and corresponding to the sequence SEQ ID NO:5.

As used herein, the expression a "mutated Hly linking segment" refers to a Hly linking segment comprising at least one amino acid modification in its sequence -i.e. as compared to its wild type sequence (e.g. SEQ ID NO:5)-, preferably between residues 37 to 107 of its sequence -i.e. as compared to SEQ ID NO:5 and corresponding to residues 410 to 480 of SEQ ID NO: 1 SEQ ID NO:2 or SEQ ID NO:3 -; and still preferably between residues 71 to 81 of its sequence -i.e. as compared to SEQ ID NO:5 and corresponding to residues 444 to 454 of SEQ ID NO:1, SEQ ID NO:2 or SEQ ID NO:3.

As used herein, the term "amino acid modification" refers to amino acid addition, amino acid deletion, and/or to amino acid substitution.

The sequence derived from a CyaA protein consists in a sequence derived from the Hly domain of CyaA -i.e. excluding a part of the AC domain - and corresponding, for example, to the amino acids 372 to 1706 or 374 to 1706 of SEQ ID NO:1, SEQ ID NO:2 or SEQ ID NO:3. In a preferred embodiment, a mutated Hly linking segment refers to a Hly linking segment comprising one or more amino acid substitutions in its sequence. Preferably, said one or more amino acid substitutions refer to the substitution of negatively charged residues -i.e. Glutamate (E) or Aspartate (D)- by another residue such as neutral residues -i.e. Asparagine (N), Glutamine (Q), Serine (S), Threonine (T), or Tyrosine (Y)- or positively charged residues -i.e. Lysine (K), Arginine (R), or Histidine (H)-; still preferably said one or more amino acid substitutions refer to negatively charged residues substitution by neutral residues. In a still preferred embodiment, the mutated Hly linking segment comprising at least one substitution at residue 445(D), 446(D) or 448(E) (numbering with respect to SEQ ID NO:1, SEQ ID NO:2 or SEQ ID NO:3). In an even more preferred embodiment the mutated Hly linking segment comprises at least one substitution selected from the group consisting of the residue 445 by an Asparagine (D445N) and by a Serine (D445S), of the residue 446 by an Asparagine (D446N) and by a Serine (D446S) and of the residue 448 by a Glutamine (E448Q) and by a Serine (E448S). As an example, the mutated Hly linking segment comprises the substitution D445N, D446N and E448Q. Still preferably, the sequence derived from a CyaA protein consists in a SEQ ID NO:8.

As used herein, the term "fragment", refers to a sequence of at least 500 consecutive amino acids, preferably of at least 1000 consecutive amino acid residues.

As used herein, the term "derived from" or "derivative" refers to an amino acid sequence having a percentage of identity of at least 75% with the amino acid sequence of a CyaA protein (i.e. a wild-type CyaA such as SEQ ID NO:1, SEQ ID NO:2 or SEQ ID NO:3) or a fragment thereof, preferably of at least 79%, at least 80%, or at least 85%, still preferably of at least 90%, at least 95%, at least 96%, at least 97%, at least 98%, or at least 99% of identity.

As used herein, "percentage of identity" between two amino acids sequences, means the percentage of identical amino-acids, between the two sequences to be compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously aligned according to the best alignment; this comparison is realized on segments of comparison in order to identify and compare the local regions of similarity. The best sequences alignment to perform comparison can be realized, beside by a manual way, by using the local homology algorithm developed by Smith and Waterman (Ad. App. Math., vol.2, p:482, 1981), by using the global homology algorithm developed by Neddleman and Wunsch (J. Mol. Biol., vol.48, p:443, 1970), by using the method of similarities developed by Pearson and Lipmolan (Proc. Natl. Acad. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (EdgarC., Nucleic Acids Research, vol. 32, p:1792, 2004). To get the best local alignment, one can preferably use the BLAST software with the BLOSUM 62 matrix. The identity percentage between two sequences of amino acids is determined by comparing these two sequences optimally aligned, the amino acids sequences being able to encompass additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.
Thus, without considering the mutation(s) in the Hly linking segment, the sequence derived from an adenylate cyclase toxin (CyaA) protein may have a percentage of identity of at least 95%, or even 100% with the amino acid sequence of a CyaA protein (i.e. a wild-type CyaA such as SEQ ID NO:1, SEQ ID NO:2 or SEQ ID NO:3).

Now, the polypeptide from the composition of the invention may also be post-translationally modified. As an example, said polypeptide can be modified by post-translational acylation of at least one of its residues, in particular at least one of the two, preferably the two, lysine residues corresponding to the residues located in positions 860 and 983 of SEQ ID NO:1 CyaA protein. By "acylation", it is meant herein palmitoylation, i.e., addition of palmitate and/or palmitoleate group(s) on such residue(s). Thus, polypeptide of the invention bears a palmitoyl group on some of these residues, preferably on one of the two, or the two, lysine residues corresponding to the residues 860 and 983 of SEQ ID NO:1, SEQ ID NO:2 or SEQ ID NO:3. By "corresponding to", it is meant that the residue(s) which is (are) post-translationally modified in the polypeptide of the invention has (have) the position(s) which matches the lysines 860 and 983 in the sequence of CyaA of SEQ ID NO:1, SEQ ID NO:2 or SEQ ID NO:3. The identification of these lysine residues in the polypeptides of the invention can be carried out by the person skilled in the art, for example by mass spectrometry.

In a second embodiment, the polypeptide as defined above comprising a sequence derived from a CyaA protein or a fragment thereof is fused to a heterologous sequence This fusion is a covalent fusion through a peptide bond. Such fusions are engineered on the level of the polypeptide-encoding DNA sequence by genetic fusion of two polypeptide-encoding DNA segments.

By " heterologous sequence", it is meant that this sequence is without any identical residues (100% identity) over more than 7 consecutive amino acid residues that originate from a CyaA protein (e.g. SEQ ID NO:1, SEQ ID NO:2 or SEQ ID NO:3). Therefore, said sequence is said heterologous, if it is not identical and/or has an identity which is less than 50%, less than 40%, less than 30%, less than 20% or even less than 10% of identity with a part of SEQ ID NO:1, SEQ ID NO:2 or SEQ ID NO:3 having the same size.

Identity being calculated for this definition by comparing this heterologous sequence and the sequence derived from a CyaA protein.

In a particular embodiment, the heterologous sequence has a size ranging from 9 to 500 amino acid residues, in particular 9 to 400 residues, 9 to 300 residues, 9 to 200 residues, 9 to 100 residues, 20 to 500 residues, 20 to 400 residues, 20 to 300 residues, 20 to 200 residues, 20 to 100 residues, 50 to 500 residues 50 to 400 residues, 50 to 300 residues, 50 to 200 residues, 50 to 100 residues, 100 to 500 residues, 100 to 400 residues, 100 to 300 residues or 100 to 250.

In a particular embodiment, said heterologous sequence comprises or consists of one or several antigen(s), each antigen comprising one or several epitope(s) as defined herein. Within the present invention, an antigen is defined as a peptidic sequence which is able to elicit an immune response, in particular a T cell immune response, against one or several epitopes contained in this sequence. An antigen is either a full-length antigenic peptidic sequence of cellular or viral origin, a fragment of this sequence able to elicit an immune response, in particular a T cell immune response, against an antigenic determinant contained in this fragment, or a synthetic, non-natural sequence which carries epitope(s) consisting of several parts of the antigenic peptidic sequence fused together, provided that the synthetic polypeptide comprising this non-natural sequence is able to elicit a T cell immune response, against an antigenic determinant contained in this sequence.

Thus, said heterologous sequence bears, comprises or consists of at least one epitope(s), preferably at least one CD8(+) epitope(s) and/or at least one CD4(+) epitope(s). By "at least", it is meant one or a plurality of epitopes. An epitope is defined herein as any amino acid sequence involved in the elicitation or induction of a cell-mediated immune response, especially a T cell immune response, and is either linear or conformational. Accordingly, epitopes described herein include those which are processed by APC (Antigen Presenting Cells) in a host, especially T epitopes recognized in association with class I MHC (Major Histocompatibility Complex) molecules, such as epitopes the target cells of which are CD8(+) T lymphocytes, or T epitopes recognized in association with class II MHC molecules, such as those which target cells are CD4(+) T lymphocytes. Epitopes within the present invention have preferably a size ranging from 9 to 17, preferably 9 to 12, residues. Epitopes described herein include also B epitopes involved in humoral response.

In a particular embodiment, said heterologous sequence comprises or consists of at least one antigen or at least one epitope, said at least one antigens or at least one epitope being of cellular origin. Thus, said at least one antigen or at least one epitope originates from a prokaryote or eukaryote cell.

In an embodiment, said at least one antigen or at least one epitope originates from a virus, a bacterium, a fungus or a parasite, such as, but not limited to, Bordetella, Chlamydia, Plasmodium, Candida, Leishmania, Leptospirosis , Mmycobacterium tuberculosis, parvovirus , distemper virus, adenovirus , parainfluenza virus , influenza virus, coronavirus, Herpesvirus, calicivirus or panleukopenia Virus.

In a particular embodiment, when the heterologous sequence comprises or consists of more than one epitope or more than one antigen, these epitopes or antigens originate from the same virus, the same bacterium, the same fungus or the same parasite.
In another particular embodiment, when the heterologous polypeptide comprises or consists of more than one epitope or more than one antigen, these epitopes or antigens originate from different organisms, such as different viruses, different bacteria, different fungi or different parasites.

In another embodiment, said at least one antigen or at least one epitope originates from mammalian cell. In a particular embodiment, said at least one antigen or at least one epitope is from a tumour antigen -i.e., a polypeptide expressed by tumour or by cancerous cells-, the tumour being self or induced by a pathogen. In a particular embodiment, the tumour antigen is self, preferably of human origin.

The term "tumour antigen" encompasses the following groups of tumour antigens, and the heterologous polypeptide contained in the chimeric protein of the invention may be chosen in at least one of the following groups: (a) oncofetal tumour antigens, (b) oncoviral tumour antigens, (c) overexpressed/accumulated tumour antigens, expressed in a wide variety of normal tissues and overexpressed in tumours, (d) shared tumour-specific antigens or cancer-Testis antigens, expressed in many tumours but not in normal tissues (including BAGE family, GAGE family, MAGE family, SAGE family and XAGE family), (e) lineage-restricted tumour antigens, (f) mutated tumour antigens, resulting from point mutations in genes that are ubiquitously expressed; and (g) differentiation tumour antigens, expressed in the normal tissue of origin of the tumours but which are not tumour-specific.

When a heterologous sequence encodes several antigens, these antigens are either fused or separated by peptide linkers or at least two of said antigens are fused whereas at least two of said antigens are separated by a linker. In a particular embodiment, said peptide linker has a size ranging from 2 to 10 residues. The linkers may be added to separate antigens and/or to improve immune,response.

The expression "fused to" when reference is made to a peptidic sequence means that each peptidic part (e.g., the sequence derived from a CyaA protein and the heterologous sequence) are covalently linked together by a peptide bond. The order of these different peptidic parts is described herein as from N-terminal to C-terminal, i.e., the last C-terminal residue of a part is linked to the first N-terminal residue of the other part by a peptide bond.

As used herein, the term" active agents", relates to an immunogenic active component in that it resembles a disease-causing pathogen or infectious agent, and/or is made from attenuated, weakened, killed and/or recombinant forms of the same, its cells, tissues, DNA, RNA, toxins, proteins subunits, particles, and/or one of its surface proteins, tumor antigens , and combination thereof such that it provokes an immune response to that pathogen or infectious agent.

The active agent may comprise a killed, but previously virulent, microorganism that has been destroyed. Such active agents are for example , but not limited to influenza, cholera, polio, hepatitis A, and rabies vaccines.
Other active agents may contain living attenuated microorganisms and/or modified living virus, which either use living active agent that have been cultivated under conditions that disable their virulent properties, or use closely related but less dangerous organisms to produce a broad immune response. Examples include, but are not limited to , yellow fever, measles, mumps, rubella, whooping cough, porcine reproductive and respiratory syndrome (PRRS), distemper, canine adenovirus Type 2, parainfluenza, and kennel cough (e.g., coronavirus) vaccines.
Some active agents are also bacterial or protozoan parasite in nature, such as, but not limited to, *Mycoplasma hyopneumoniae, Bordetella bronchiseptica, Bordetella pertusis, Mycobacterium tuberculosis, Salmonella typhi* (typhoid), *Staphylococcus aureus, Staphylococcus pseudintermedius, Leptospira interrogans, Leptospira grippotyphosa, Leptospira Pomona, Leptospira spirochete, Leishmania* and *Streptococcus suis.*

Other suitable active agents include toxoids made from inactivated toxic compounds that cause illness rather than the microorganism itself. Such examples are, but not limited to, Cryptosporidiosis, neosporosis, toxoplasmosis, tetanus and diphtheria toxoid-based vaccines. Protein subunit active agents can also be used, in which a fragment of the microorganism is used to create an immune response. Examples include , but are not limited to, subunit vaccines against HPV, hepatitis B, and the hemagglutinin and neuraminidase subunits of the influenza virus.
Active agents can also be formulated using viral or bacterial DNA to provoke an immune response. Furthermore, although most current vaccines are created using inactivated or attenuated compounds from microorganisms, synthetic active agents using synthetic peptides, carbohydrates, or antigens can also be used.
Cancer vaccines or immunotherapies using tumor antigens as the active agent are also contemplated herein. Suitable immunogenic compositions can be monovalent or polyvalent.

In a preferred embodiment, the at least one active agent and the polypeptide heterologous sequence here above defined are antigens from the same pathogen. As an illustration, for example, and without restriction, the active antigen can be an inactivated Bordetella bacteria and the heterologous sequence fused with adenylate cyclase toxin (CyaA) is a fragment of a self-associating tip complex protein from the type III secretion system (T3SS) from Bordetella such as Bsp 22 (e.g. *Bordetella bronchiseptica,* accession number WP003809882.1) or the P36 protein from *Leishmania infantum* (Accession number XP001470319.2, SEQ ID NO :7).

The immunogenic composition of present invention eventually also comprises a suitable pharmaceutical vehicle, which is, for example ,selected from buffering agents, saline, phosphate buffered saline, dextrose, glycerol, water, ethanol and the like, stabilizers, preservatives, adjuvant, surfactants and/or immunomodulatory substances (such as cytokines or chemokines) and/or growth factors such as GM-CSF and combinations thereof.

Stabilizers are used to help the immunogenic composition to maintain its effectiveness during storage. The composition stability is essential, particularly where the cold chain is unreliable. Stabilizing agents include for example , but not limited to, MgCl2 MgSO4, lactose-sorbitol and sorbitol-gelatin.

Preservatives are added to the immunogenic composition to prevent bacterial and fungal growth. They include a variety of substances, for example, but not limited to antibiotics, thiomersal, formaldehyde, or phenol derivatives.

Adjuvants are known in the art and include for example , but not limited to , Complete Freund's Adjuvant (CFA), Incomplete Freund's Adjuvant (IFA), montanide ISA (incomplete seppic adjuvant), muramyl peptides such as muramyl dipeptide (MDP) MDP-Lys (L18) (N.sup..alpha.-acetylemuramyl-L-alanyl-D-isoglutaminyl-N.sup,esteoroyl-L-- lysine), zinc sulphate, colloidal iron hydroxide, calcium phosphate or calcium chloride, CpG oligodeoxynucleotides (CPG ODN) such as CPG ODN 1826 and CPG ODN 2007, MF59 which is a detergent stabilized oil-in water emulsion containing 5% squalene (w/v), 0.5% Tween™ 80 (w/v) and 0.5% Span (w/v) in water, TLR4 ligands (such as MPL, GLA) TLR3 ligands (such as Poly IC, Poly-ICLC called Hiltonol™.), polysaccharides (such as Insulin) and liposomes (such as cationic liposomes, ISCOMs).

In a particular embodiment, at least one adjuvant is chosen among molecules which have the capacity to activate T-cell immune response. Preferred adjuvants are the ones that bind or are agonist to PRR (Pathogen Recognition Receptors) and even more preferably to TLR (Toll like receptor) 3, 4, 7, 8 and/or 9 on immune cells (such as APC). In a particular embodiment, the adjuvant is a TLR ligand, in particular a TLR ligand selected from the group consisting of TLR ligands of class 3, such as poly-ICLC, TLR ligands of class 4, such as MPL TLR ligands of class 9, such as CpG, and TLR ligands of class 7/8, such as Imiquimod. Examples of adjuvants are Imiquimod and Poly-ICLC. A commercially available drug based on Imiquimod is Aldara™ (sold as a cream containing 5% Imiquimod) Poly-ICLC can be purchased from Oncovir Inc, (WA, US) as Hiltonol™.

The immunogenic composition of the invention can be injected in a human and/or non human animal by different routes: subcutaneous (s.c.), intradermal (i.d.), intramuscular (i.m.) or intravenous (i.v.) injection, oral administration and mucosal administration, especially intranasal administration or inhalation. In a particular embodiment, immunogenic composition(s) defined herein is/are administered intradermally.

The immunogenic composition of the invention may be in a solid form (capsule, powder, tablet, pill, suppository, quick release tablet, gastro-resistant tablet, delayed release tablet), a powder form, preferably after lyophilization (lyophilized form or lyophilized powder form) which needs to be reconstituted for example with diluents(s) before injection, or in a liquid form, such as an injectable solution or injectable suspension.

The immunogenic composition of the invention is for use in the prophylaxis and/or treatment of a pathogen infection. In another embodiment, the immunogenic composition of the present invention is for use in the prophylaxis and/or treatment of an oncogenic based disorder.

In a particular embodiment, the invention also relates to a method for the therapeutic treatment of a human and/or a non human animal presenting a pathogen infection or suspected to be infected with a pathogen comprising (a) the administration of an effective amount of an immunogenic composition of the present invention into said human and/or non human animal, possibly as multiple administered doses, and (b) the follow up of the condition of said human and/or non human animal.

The invention also relates to a method to prevent a pathogen infection of a human and/or of a non human animal comprising (a) the administration of an effective amount of an immunogenic composition of the present invention into said human and/or non human animal, possibly as multiple administered doses, and (b) the follow up of the condition of said human and/or non human animal, possibly as multiple administered doses.

In another embodiment, the invention also relates to a method for the therapeutic treatment of a human and/or of a non human animal suffering from tumor disorders comprising (a) the administration of an effective amount of an immunogenic composition of the present invention into said human and/or non human animal, possibly as multiple administered doses, and (b) the follow up of the condition of said human and/or non human animal.

An "effective amount" of the immunogenic composition of the invention meant that is sufficient to prevent or inhibit the pathogen infection or the growth of cancer cells. The doses used for the administration can be adapted as a function of various parameters, in particular as a function of the mode of administration used, of the relevant pathology; or alternatively of the desired duration of treatment. Naturally, the form of the immunogenic composition, the route of administration, the dosage and the regimen depend on the condition to be treated, the severity of the illness, the age, weight, and sex of the subject, etc.

For example, the amount of active agent present in the immunogenic compositions of the present invention will vary depending upon the particular active agent used, as well as the intended subject for administration of the composition, and route of administration... In general, the immunogenic composition of the present invention will comprise from about 0.01% to about 90% by weight of the active agent, preferably from about 10% to about 60% and more preferably, from about 30% to about 50%, based upon the total weight of the immunogenic composition taken as 100% by weight.

The immunogenic compositions will also comprise from about 0.5% to about 80% by weight of the polypeptide disclosed previously or of the polynucleotide encoding thereof, preferably from about 10% to about 60% by weight and more preferably, from about 30 to about 50%, based upon the total weight of the immunogenic composition taken as 100% by weight.

In some embodiments, the weight ratio of the polypeptide or the nucleic acid encoding thereof, to active agent in the immunogenic composition will be from about 1: 1 to about 50:1, preferably from about 10: 1 to about 30: 1, and more preferably from about 20: 1 to about 40: 1.

The ranges of effective doses provided below are not intended to limit the invention and represent preferred dose ranges. However, the preferred dose can be tailored to the individual subject, as is understood and determinable by one of skill in the art, without undue experimentation.

A therapeutic treatment according to the invention aims at improving the clinical condition of a human and/or a non human animal in need thereof, who has been diagnosed as being infected or suspected to be infected by a pathogen or as suffering from a pathological state. In a particular embodiment, this treatment aims at the elimination of the causative agent or organism of the disease, or at lowering the abundance of said agent or organism. In a situation of viral infection, the treatment may result in a significant decrease of the viral load in the targeted tissues of the host that is less than what can be detected when measured. In case of tumoral disorders, the treatment may result at lowering the size or the development of the tumour(s), or at eradicating the tumour cells, or at reducing the number of tumour cells at a level which is less than what can be detected when measured. The therapeutic treatment also aims at improving the clinical status of the human and/or non human animal, by eliminating or lowering the symptoms associated with the pathogen infection or the tumour disorders, and preferably aims at restoring health.

A prophylactic treatment of a human and/or a non human animal aims at preventing the pathogen infection of said human and/or non human animal, or preventing the apparition or development of neoplastic tumoral disorders, or preventing the occurrence of a pathological state in said human and/or non human animal. The prophylactic treatment encompasses vaccination.

Therapeutic and prophylactic treatments, using a composition of the invention, are based on the elicitation of an efficient immune response, preferably a cellular immune response, against the epitope(s) contained in the heterologous polypeptide in the host.

The term "non human animal" encompasses, and preferably is a non human mammal such as for example a canine, a feline, a bovine, an ovine, a porcine, a camelid and/or an equine.

The present invention will be understood more clearly on reading the description of the experimental studies performed in the context of the research carried out by the applicant, which should not be interpreted as being limiting in nature.

### EXAMPLES

### Example 1. Construction, production and purification of CyaA protein.

*Antigens :* The synthetic peptides corresponding to antigens such as Bsp22 of *Bordetella Bronchiseptica* (Accession number WP003809882.1, SEQ ID NO:6) and *Leishmania infantum* P36 protein (Gonzales-Aseguinolaza et al., Eur. J. Biochem. 259: 909-916, Accession number XP001470319.2, SEQ ID NO :7) were purchased from Polypeptide (Strasbourg, France). *Construction of recombinant CyaA protein:* Recombinant CyaA protein comprising the antigens such as Bsp22 (CyaA-Bsp22, SEQ ID NO:9) or *Leishmania infantum* P36 (CyaA-P36, SEQ ID NO:10) genetically link to the N- terminal part of *Bordetella Bronchiseptica* CyaA protein which is the polypeptide from residues 374 to 1706.

These recombinant CyaA proteins were produced in E. coli XL1 Blue (Stratagen, an Agilent Technologies Company, USA) transformed with appropriate pT7CACT1-derived constructs as in described in Osicka R. et al. (2000, Infect. Immun., 68: 247-256). Exponential 500-ml cultures were grown at 37°C and induced by isopropyl 1-thio-β-D-galactopyranoside (IPTG, 1 mM) for 4 h before the cells were washed with 50 mM Tris-HCl (pH 8.0), 150 mM NaCl, resuspended in 50 mM Tris-HCl (pH 8.0), 0.2 mM CaCl2, and disrupted by sonication. Upon centrifugation at 25,000 x g for 20 min, the insoluble cell pellets were resuspended in 8 M urea, 50 mM Tris-HCl (pH 8.0), 50 mM NaCl, 0.2 mM CaCl2. Upon centrifugation at 25,000 x g for 20 min, clarified urea extracts were loaded onto a DEAE-Sepharose column equilibrated with 8 M urea, 50 mM Tris-HCl (pH 8.0), 120 mM NaCl. After washing, the CyaA proteins were eluted with 8 M urea, 50 mM Tris-HCl (pH 8.0), 2 M NaCt, diluted four times with 50 mM Tris-HCl (pH 8.0), 1 M NaCl buffer, and further purified on a phenyl-Sepharose column equilibrated with the same buffer. Unbound proteins were washed out with 50 mM Tris-HCl (pH 8.0), and the CyaA proteins were eluted with 8 M urea, 50 mM Tris-HCl (pH 8.0), 2 mM EDTA and stored at -20°C. Concentrations of the purified CyaA proteins were determined by the Bradford assay (Bio-Rad, Hercules, USA) using bovine serum albumin as a standard. All purified CyaA recombinant proteins were more than 90% pure as judged by SDS-gel analysis.

### Example 2. Bordetella Bronchiseptica infection

### Bacterial strains and infection procedure

*Bordetella bronchiseptica* strains were purchased from the Pasteur Institute Collection (CRBIP4.201 and CRBIP4.202) and ATTC (Bb 31124 Moreno-Lopez). Strains were inoculated on Bordet Gengou agar plates supplemented with 15% defibrinated sheep blood (Biomerieux, France) and incubated aerobically for 48 hours at 37°C. Strains were then transferred to Stainer-Scholte broth and incubated, with shaking, under aerobic conditions for 18 hours at 37°C. The cultures were stopped when the DO at 600nm reached 1. The bacterial count at D.O.=1 was determined to be 10⁹ cfu/ml. The challenge preparation was an equal mix of the three bacterial strains, corresponding to a total of 8.0 10⁹CFU/ml.

### I. Mice Bordetella Bronchiseptica infection challenge

Six week old female BALB/c mice (Janvier Labs, France) were immunized with two doses of subcutaneous Injections in the upper back at 3-weeks intervals of either 250µl (¼ of a canine dose of Bronchicine Cae, Zoetis) (Group 1), 250µl (¼ of a canine dose of Bronchicine Cae, Zoetis) plus 75µg of CyaA-Bsp22 as described in Example 1 (SEQIDNO: 9)(in Urea 2.4M) (Group 2) and 2.4M Urea for control mice (Group 3).

Three weeks after the immunization, 12 mice per group were challenged by instillation into the left nostril under ether anesthesia of 50µl of *Bordetella Bronchiseptica* suspension as defined hereabove and equivalent to a total of approximately 2x10⁸ colony forming units [CFU]). The survival curve was determined with the Kaplan Meier method.

### Results :

At a dose of 2 x 10⁸ CFU/mouse, we observed a mortality rate of 90% in control group seven days after the infection. This mortality rate was significantly decreased in mice vaccinated with Bronchicine Cae. Interestingly, immunization with a combination of Bronchicine Cae plus CyaA-Bsp22 resulted in no significant mortality in this group of mice.

### ii. Dogs Bordetella Bronchiseptica infection challenge

Beagle dogs were purchased from a licensed kennel for animal breeding and husbandry (CEDS, Centre d'Elevage du Domaine de Souches, France). Specific pathogen-free puppies were eight weeks old at their arrival. They were bred in a specific facility free of *Bordetella bronchispetica.* Before the beginning of the study, all the puppies were shown to be seronegative for *Bordetella bronchiseptica.* All dogs were dewormed and vaccinated against canine distemper virus, parvovirus, leptospirosis, infectious canine hepatitis and parainfluenza virus. They were housed in an accredited class II facility and were provided commercial dog food (VetCare Nutrition, Junior 10kg, Royal Canin) and tap water ad libitum.

In total, 25 dogs were used, 13 females and 12 males. Dogs were randomly allocated to two groups, following an equivalent ratio of male vs female in each group. The control group was composed of 5 puppies and the vaccinated group composed of 20 puppies divided in two groups: one group of 10 puppies received two doses of canine Bronchicine Cae as recommended by the manufactor Zoetis (Group 1) and a group of 10 puppies received the same dose of canine Bronchicine Cae plus 300 µg of CyaA-Bsp22 as described in Example 1 (in Urea 2.4M) (Group 2).

All dogs received two parenteral vaccine injections at 10 weeks of age (2 weeks after their arrival) and three weeks later. The control group received the same amount of excipient and adjuvant as in the vaccine formulation.

All dogs were infected by *Bordetella bronchiseptica* four weeks after the second vaccine injection. All puppies were exposed to an aerosol of 1 ml of challenge preparation (8 x10⁹ CFU/ml during 15 min using a nebuliser (OMRON, compAir Pro C900). Puppies were slightly tranquilized with an intramuscular injection of a mix of butorphenol (0.3mg/kg) and acepromazine (0.01mg/kg) 10 min before the aerosol nebulization.

### Clinical follow-up

A clinical follow-up was performed daily from the day before challenge and during three weeks post infection following the criteria presented in table 1.

**Table 1: Clinical follow-up post infectious challenge**

| Rectal Temperature (°C) : | | |
|---|---|---|
| Weight (kg): | | |
| | Qualifier | |
| Lethargy | | |
| Cough | Absente | |
| | Induced | |
| | Spontaneous | |
| | | |
| Nasal Discharge | Clear | |
| | Thick | |
| Sneezing | Frequent | |
| Vomiting | | |
| Dyspnea | | |

### Results:

Dogs in the control group (Group 3) showed signs of severe respiratory disease and had a cumulative average score of 6.
Dogs in the Group 1 (Subcutaneous vaccine) showed signs of spontaneous, harsh cough that continued for several days and had average cumulative score of 3.
Dogs in Group 2 (Subcutaneous vaccine plus CyaA-Bsp22) showed very few signs of disease post challenge with no further complications.

### Example 3. Leishmania infection challenge

Six week old female BALB/c mice (Janvier Labs, France) were immunized with three doses of subcutaneous Injections in the upper back at 21 days of intervals of either 250µl (¼ of a canine dose of CaniLeish, Virbac) (Group 1) ,250µl (¼ of a canine dose of CaniLeish, Virbac) plus 75µg of CyaA- P36 as obtained in Example 1 (in Urea 2.4M) (Group 2) and 2.4M Urea for control mice (Group 3).

Three weeks after the immunization, 10 mice per group were challenged intradermally in their left hind footpad with 1 10⁷ stationnary phase promastigotes of *L. infantum* (strain MHOM/BR/2001/HP-EMO or strain MHOM/BR/1970/BH46). Ten weeks after infection, animals were euthanized and spleen, liver, infected pawsi's draining lymph nodes (dLN) and bone marrow (BM) were collected to evaluate the parasite burden and visceral lesions in the animals.

The liver, spleen, dLN and BM were collected for parasite quantification. The limiting-dilution technique was used according to the technique previously described (Martins et al., 2013).

### Results:

As a measure of protection, we compared lesion development and parasite burden after intradermal challenge with live *L. infantum* 10 weeks after infection. The greatest degree of protection was observed in mice given the combination of CaniLeish vaccine and CyaA-P36 toxoid, with a significant decrease of parasite burden and a significant reduction of visceral leishmaniosis.

## Claims

1. An immunogenic composition comprising :
i) a polypeptide comprising a sequence derived from an adenylate cyclase toxin (CyaA) protein or a fragment thereof, or a polynucleotide encoding thereof, and
ii) at least one active agent.

2. The immunogenic composition of claim 1, wherein said active agent is selected in the group comprising or consisting of attenuated, weakened, killed, recombinant forms, cells, tissues, DNA, RNA, toxins, proteins subunits, particles, and/or surface proteins to a pathogen, an infectious agent or a tumor and combination thereof.

3. The immunogenic composition of claim 2 where the pathogen or the infectious agent is from bacterial, parasitic, viral, or fungal origin.

4. The immunogenic composition of claim 3 where said bacteria is *Mycoplasma hyopneumoniae, Bordetella bronchiseptica, Bordetella pertusis, Mycobacterium tuberculosis, Salmonella typhi, Staphylococcus aureus, Leptospira interrogans, Leptospira grippotyphosa, Leptospira Pomona, Leptospira spirochete, Leishmania* and *Streptococcus suis.*

5. An immunogenic composition of claim 1, wherein the polypeptide further comprises a heterologous sequence which is fused to the sequence derived from a CyaA protein or fragment thereof.

6. The immunogenic composition of claim 1, wherein the sequence derived from an adenylate cyclase toxin (CyaA) comprises a deletion in the amino-terminal adenylate cyclase (AC) domain.

7. The immunogenic composition of claim 6 wherein said deletion in the AC domain refers to a deletion of at least 100 amino acids, preferably of at least 200 amino acids; and most preferably of at least 300 amino acids in the AC domain.

8. The immunogenic composition of claim 7 wherein said polypeptide comprises a mutated Hly linking segment and preferably at least one substitution at residue 445(D), 446(D) or 448(E) (numbering with respect to SEQ ID NO:1, SEQ ID NO:2 or SEQ ID NO:3).

9. The immunogenic composition of claim 8 wherein the sequence derived from an adenylate cyclase toxin (CyaA) has the sequence SEQ ID NO:8.

10. An immunogenic composition wherein the heterologous sequence consists in Bsp 22 from *Bordetella bronchiseptica* (SEQ ID NO:6) or in the P36 protein from *Leishmania infantum* (SEQ ID NO :7), or fragments thereof.

11. A composition as defined in any one of claims 1 to 10 for use in the prophylaxis and/or treatment of a pathogen infection.


SEQUENCE LISTING

<110> CYANIMAL IP
X, X

<120> CyaA polypeptides as immune enhancer

<130> CYA-B-0002-EP1

<160> 10

<170> PatentIn version 3.5

<210> 1
<211> 1706
<212> PRT
<213> Bordetella pertussis

<400> 1

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Ser Gly Arg Ser Lys Phe Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala
        675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
            740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Val
785                 790                 795                 800

Asp Val Phe Val Asp Arg Phe Val Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Asp Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Thr Thr Ala Tyr Gly Lys Arg Thr Glu Asn Val Gln Tyr Arg His
            980                 985                 990

Val Glu Leu Ala Arg Val Gly Gln Leu Val Glu Val Asp Thr Leu Glu
        995                 1000                1005

His Val Gln His Ile Ile Gly Gly Ala Gly Asn Asp Ser Ile Thr
    1010                1015                1020

Gly Asn Ala His Asp Asn Phe Leu Ala Gly Gly Ser Gly Asp Asp
    1025                1030                1035

Arg Leu Asp Gly Gly Ala Gly Asn Asp Thr Leu Val Gly Gly Glu
    1040                1045                1050

Gly Gln Asn Thr Val Ile Gly Gly Ala Gly Asp Asp Val Phe Leu
    1055                1060                1065

Gln Asp Leu Gly Val Trp Ser Asn Gln Leu Asp Gly Gly Ala Gly
    1070                1075                1080

Val Asp Thr Val Lys Tyr Asn Val His Gln Pro Ser Glu Glu Arg
    1085                1090                1095

Leu Glu Arg Met Gly Asp Thr Gly Ile His Ala Asp Leu Gln Lys
    1100                1105                1110

Gly Thr Val Glu Lys Trp Pro Ala Leu Asn Leu Phe Ser Val Asp
    1115                1120                1125

His Val Lys Asn Ile Glu Asn Leu His Gly Ser Arg Leu Asn Asp
    1130                1135                1140

Arg Ile Ala Gly Asp Asp Gln Asp Asn Glu Leu Trp Gly His Asp
    1145                1150                1155

Gly Asn Asp Thr Ile Arg Gly Arg Gly Gly Asp Asp Ile Leu Arg
    1160                1165                1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Pro
    1205                1210                1215

Gly Arg Ile Val Ala Pro His Glu Tyr Gly Phe Gly Ile Glu Ala
    1220                1225                1230

Asp Leu Ser Arg Glu Trp Val Arg Lys Ala Ser Ala Leu Gly Val
    1235                1240                1245

Asp Tyr Tyr Asp Asn Val Arg Asn Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Gln Ala Arg
    1325                1330                1335

Glu His Asp Val Leu Arg Gly Gly Asp Gly Val Asp Thr Val Asp
    1340                1345                1350

Tyr Ser Gln Thr Gly Ala His Ala Gly Ile Ala Ala Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Asp Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Arg Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg Asn Ile Glu Asn Ala Val Gly Ser Ala Arg Asp
    1520                    1525                    1530

Asp Val Leu Ile Gly Asp Ala Gly Ala Asn Val Leu Asn Gly Leu
    1535                    1540                    1545

Ala Gly Asn Asp Val Leu Ser Gly Gly Ala Gly Asp Asp Val Leu
    1550                    1555                    1560

Leu Gly Asp Glu Gly Ser Asp Leu Leu Ser Gly Asp Ala Gly Asn
    1565                    1570                    1575

Asp Asp Leu Phe Gly Gly Gln Gly Asp Asp Thr Tyr Leu Phe Gly
    1580                    1585                    1590

Val Gly Tyr Gly His Asp Thr Ile Tyr Glu Ser Gly Gly Gly His
    1595                    1600                    1605

Asp Thr Ile Arg Ile Asn Ala Gly Ala Asp Gln Leu Trp Phe Ala
    1610                    1615                    1620

Arg Gln Gly Asn Asp Leu Glu Ile Arg Ile Leu Gly Thr Asp Asp
    1625                    1630                    1635

Ala Leu Thr Val His Asp Trp Tyr Arg Asp Ala Asp His Arg Val
    1640                    1645                    1650

Glu Ile Ile His Ala Ala Asn Gln Ala Val Asp Gln Ala Gly Ile
    1655                    1660                    1665

Glu Lys Leu Val Glu Ala Met Ala Gln Tyr Pro Asp Pro Gly Ala
    1670                    1675                    1680

Ala Ala Ala Ala Pro Pro Ala Ala Arg Val Pro Asp Thr Leu Met
    1685                    1690                    1695

Gln Ser Leu Ala Val Asn Trp Arg
    1700                    1705

<210>  2
<211>  1706
<212>  PRT
<213>  Bordetella bronchiseptica

<400>  2

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr

35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asn Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Pro Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly

305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Gly Gly Arg Ser Lys Ser Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala

            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala
        675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
            740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Ala
785                 790                 795                 800

Asp Val Phe Val Asp Arg Phe Ile Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr

850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Glu Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Lys Thr Ala Tyr Gly Lys Arg Thr Glu Asn Val Gln Tyr Arg His
            980                 985                 990

Val Glu Leu Ala Arg Val Gly Gln  Leu Val Glu Val Asp  Thr Leu Glu
        995                 1000                 1005

His Val  Gln His Ile Ile Gly  Gly Ala Gly Asn Asp  Ser Ile Thr
    1010                 1015                 1020

Gly Asn  Ala His Asp Asn Phe  Leu Ala Gly Gly Ala  Gly Asp Asp
    1025                 1030                 1035

Arg Leu  Asp Gly Gly Ala Gly  Asn Asp Thr Leu Val  Gly Gly Glu
    1040                 1045                 1050

Gly His  Asn Thr Val Val Gly  Gly Ala Gly Asp Asp  Val Phe Leu
    1055                 1060                 1065

Gln Asp  Leu Gly Val Trp Ser  Asn Gln Leu Asp Gly  Gly Ala Gly
    1070                 1075                 1080

Val Asp  Thr Val Lys Tyr Asn  Val His Gln Pro Ser  Glu Glu Arg
    1085                 1090                 1095

Leu Glu  Arg Met Gly Glu Thr  Gly Ile His Ala Asp  Leu Gln Lys
    1100                 1105                 1110

Gly Thr  Val Glu Lys Trp Pro  Ala Leu Asn Leu Phe  Ser Val Asp

1115                    1120                    1125

His Val Lys Asn Ile Glu Asn Leu His Gly Ser Ser Leu Asn Asp
    1130                    1135                    1140

Ser Ile Ala Gly Asp Asp Arg Asp Asn Glu Leu Trp Gly Asp Asp
    1145                    1150                    1155

Gly Asn Asp Thr Ile His Gly Arg Gly Gly Asp Asp Ile Leu Arg
    1160                    1165                    1170

Gly Gly Leu Gly Leu Asp Thr Leu Tyr Gly Glu Asp Gly Asn Asp
    1175                    1180                    1185

Ile Phe Leu Gln Asp Asp Glu Thr Val Ser Asp Asp Ile Asp Gly
    1190                    1195                    1200

Gly Ala Gly Leu Asp Thr Val Asp Tyr Ser Ala Met Ile His Ala
    1205                    1210                    1215

Gly Lys Ile Val Ala Pro His Glu Tyr Gly Phe Gly Ile Glu Ala
    1220                    1225                    1230

Asp Leu Ser Arg Glu Trp Val Arg Lys Ala Ser Ala Leu Gly Val
    1235                    1240                    1245

Asp Tyr Tyr Asp Ser Val Arg Asn Val Glu Asn Val Ile Gly Thr
    1250                    1255                    1260

Ser Met Lys Asp Val Leu Ile Gly Asp Ala Gln Ala Asn Thr Leu
    1265                    1270                    1275

Met Gly Gln Gly Gly Asp Asp Thr Val Arg Gly Gly Asp Gly Asp
    1280                    1285                    1290

Asp Leu Leu Phe Gly Gly Asp Gly Asn Asp Met Leu Tyr Gly Asp
    1295                    1300                    1305

Ala Gly Asn Asp Thr Leu Tyr Gly Gly Leu Gly Asp Asp Thr Leu
    1310                    1315                    1320

Glu Gly Gly Ala Gly Asn Asp Trp Phe Gly Gln Thr Pro Ala Arg
    1325                    1330                    1335

Glu His Asp Val Leu Arg Gly Gly Ala Gly Val Asp Thr Val Asp
    1340                    1345                    1350

Tyr Ser Gln Ala Gly Ala His Ala Gly Val Ala Thr Gly Arg Ile
    1355                    1360                    1365

Gly Leu Gly Ile Leu Ala Asp Leu Gly Ala Gly Arg Val Asp Lys

1370                    1375                    1380

Leu Gly  Glu Ala Gly Ser Ser  Ala Tyr Asp Thr Val  Ser Gly Ile
    1385                    1390                    1395

Glu Asn  Val Val Gly Thr Glu  Leu Ala Asp Arg Ile  Thr Gly Asp
    1400                    1405                    1410

Ala Gln  Ala Asn Val Leu Arg  Gly Ala Gly Gly Ala  Asp Val Leu
    1415                    1420                    1425

Ala Gly  Gly Glu Gly Asp Asp  Val Leu Leu Gly Gly  Glu Gly Asp
    1430                    1435                    1440

Asp Gln  Leu Ser Gly Asp Ala  Gly Arg Asp Arg Leu  Tyr Gly Glu
    1445                    1450                    1455

Ala Gly  Asp Asp Trp Phe Phe  Gln Asp Ala Ala Asn  Ala Gly Asn
    1460                    1465                    1470

Leu Leu  Asp Gly Gly Asp Gly  Asn Asp Thr Val Asp  Phe Ser Gly
    1475                    1480                    1485

Pro Gly  Arg Gly Leu Asp Ala  Gly Ala Lys Gly Val  Phe Leu Ser
    1490                    1495                    1500

Leu Gly  Lys Gly Phe Ala Ser  Leu Met Asp Glu Pro  Glu Thr Ser
    1505                    1510                    1515

Asn Val  Leu Arg His Ile Glu  Asn Ala Val Gly Ser  Val Arg Asp
    1520                    1525                    1530

Asp Val  Leu Ile Gly Asp Ala  Gly Ala Asn Val Leu  Asn Gly Leu
    1535                    1540                    1545

Ala Gly  Asn Asp Val Leu Ser  Gly Gly Ala Gly Asp  Asp Val Leu
    1550                    1555                    1560

Leu Gly  Asp Glu Gly Ser Asp  Leu Leu Ser Gly Asp  Ala Gly Asn
    1565                    1570                    1575

Asp Asp  Leu Phe Gly Gly Gln  Gly Asp Asp Thr Tyr  Leu Phe Gly
    1580                    1585                    1590

Ala Gly  Tyr Gly His Asp Thr  Ile Tyr Glu Ser Gly  Gly Gly His
    1595                    1600                    1605

Asp Thr  Ile Arg Ile Asn Ala  Gly Ala Asp Gln Leu  Trp Phe Ala
    1610                    1615                    1620

Arg Gln  Gly Asn Asp Leu Glu  Ile Arg Ile Leu Gly  Thr Asp Asp

1625                    1630                    1635

Ala Leu Thr Val His Asp Trp Tyr Arg Asp Ala Asp His Arg Val
    1640                    1645                    1650

Glu Ala Ile His Ala Ala Asn Gln Ala Ile Asp Pro Ala Gly Ile
    1655                    1660                    1665

Glu Lys Leu Val Glu Ala Met Ala Gln Tyr Pro Asp Pro Gly Ala
    1670                    1675                    1680

Ala Ala Ala Ala Pro Pro Ala Ala Arg Val Pro Asp Thr Leu Met
    1685                    1690                    1695

Gln Ser Leu Ala Val Asn Trp Arg
    1700                    1705

<210>  3
<211>  1706
<212>  PRT
<213>  Bordetella parapertussis

<400>  3
   Met Gln Gln Ser His Gln Ala Gly Tyr Ala Asn Ala Ala Asp Arg Glu
   1               5                   10                  15
   Ser Gly Ile Pro Ala Ala Val Leu Asp Gly Ile Lys Ala Val Ala Lys
               20                  25                  30
   Glu Lys Asn Ala Thr Leu Met Phe Arg Leu Val Asn Pro His Ser Thr
           35                  40                  45
   Ser Leu Ile Ala Glu Gly Val Ala Thr Lys Gly Leu Gly Val His Ala
       50                  55                  60
   Lys Ser Ser Asp Trp Gly Leu Gln Ala Gly Tyr Ile Pro Val Asn Pro
   65                  70                  75                  80
   Asn Leu Ser Lys Leu Phe Gly Arg Ala Pro Glu Val Ile Ala Arg Ala
                   85                  90                  95
   Asp Asn Asp Val Asn Ser Ser Leu Ala His Gly His Thr Ala Val Asp
               100                 105                 110
   Leu Thr Leu Ser Lys Glu Arg Leu Asp Tyr Leu Arg Gln Ala Gly Leu
           115                 120                 125
   Val Thr Gly Met Ala Asp Gly Val Val Ala Ser Asn His Ala Gly Tyr
       130                 135                 140
   Glu Gln Phe Glu Phe Arg Val Lys Glu Thr Ser Asp Gly Arg Tyr Ala
   145                 150                 155                 160
   Val Gln Tyr Arg Arg Lys Gly Gly Asp Asp Phe Glu Ala Val Lys Val
   165 170 175
   Ile Gly Asn Ala Ala Gly Ile Pro Leu Thr Ala Asp Ile Asp Met Phe
   180 185 190
   Ala Ile Met Pro His Leu Ser Asn Phe Arg Asp Ser Ala Arg Ser Ser
   195 200 205
   Val Thr Ser Gly Asp Ser Val Thr Asp Tyr Leu Ala Arg Thr Arg Arg
   210 215 220
   Ala Ala Ser Glu Ala Thr Gly Gly Leu Asp Arg Glu Arg Ile Asp Leu
   225 230 235 240
   Leu Trp Lys Ile Ala Arg Ala Gly Ala Arg Ser Ala Val Gly Thr Glu
   245 250 255
   Ala Arg Arg Gln Phe Arg Tyr Asp Gly Asp Met Asn Ile Gly Val Ile
   260 265 270
   Thr Asp Phe Glu Leu Glu Val Arg Asn Ala Leu Asn Arg Arg Ala His
   275 280 285
   Ala Val Gly Ala Gln Asp Val Val Gln His Gly Thr Glu Gln Asn Asn
   290 295 300
   Pro Phe Pro Glu Ala Asp Glu Lys Ile Phe Val Val Ser Ala Thr Gly
   305 310 315 320
   Glu Ser Gln Met Leu Thr Arg Gly Gln Leu Lys Glu Tyr Ile Gly Gln
   325 330 335
   Gln Arg Gly Glu Gly Tyr Val Phe Tyr Glu Asn Arg Ala Tyr Gly Val
   340 345 350
   Ala Gly Lys Ser Leu Phe Asp Asp Gly Leu Gly Ala Ala Pro Gly Val
   355 360 365
   Pro Gly Gly Arg Ser Lys Ser Ser Pro Asp Val Leu Glu Thr Val Pro
   370 375 380
   Ala Ser Pro Gly Leu Arg Arg Pro Ser Leu Gly Ala Val Glu Arg Gln
   385 390 395 400
   Asp Ser Gly Tyr Asp Ser Leu Asp Gly Val Gly Ser Arg Ser Phe Ser
   405 410 415
   Leu Gly Glu Val Ser Asp Met Ala Ala Val Glu Ala Ala Glu Leu Glu
   420 425 430
   Met Thr Arg Gln Val Leu His Ala Gly Ala Arg Gln Asp Asp Ala Glu
           435                 440                 445
   Pro Gly Val Ser Gly Ala Ser Ala His Trp Gly Gln Arg Ala Leu Gln
       450                 455                 460
   Gly Ala Gln Ala Val Ala Ala Ala Gln Arg Leu Val His Ala Ile Ala
   465                 470                 475                 480
   Leu Met Thr Gln Phe Gly Arg Ala Gly Ser Thr Asn Thr Pro Gln Glu
                   485                 490                 495
   Ala Ala Ser Leu Ser Ala Ala Val Phe Gly Leu Gly Glu Ala Ser Ser
               500                 505                 510
   Ala Val Ala Glu Thr Val Ser Gly Phe Phe Arg Gly Ser Ser Arg Trp
           515                 520                 525
   Ala Gly Gly Phe Gly Val Ala Gly Gly Ala Met Ala Leu Gly Gly Gly
       530                 535                 540
   Ile Ala Ala Ala Val Gly Ala Gly Met Ser Leu Thr Asp Asp Ala Pro
   545                 550                 555                 560
   Ala Gly Gln Lys Ala Ala Val Gly Ala Glu Ile Ala Leu Gln Leu Thr
                   565                 570                 575
   Gly Gly Thr Val Glu Leu Ala Ser Ser Ile Ala Leu Ala Leu Ala Ala
               580                 585                 590
   Ala Arg Gly Val Thr Ser Gly Leu Gln Val Ala Gly Ala Ser Ala Gly
           595                 600                 605
   Ala Ala Ala Gly Ala Leu Ala Ala Ala Leu Ser Pro Met Glu Ile Tyr
       610                 615                 620
   Gly Leu Val Gln Gln Ser His Tyr Ala Asp Gln Leu Asp Lys Leu Ala
   625                 630                 635                 640
   Gln Glu Ser Ser Ala Tyr Gly Tyr Glu Gly Asp Ala Leu Leu Ala Gln
                   645                 650                 655
   Leu Tyr Arg Asp Lys Thr Ala Ala Glu Gly Ala Val Ala Gly Val Ser
               660                 665                 670
   Ala Val Leu Ser Thr Val Gly Ala Ala Val Ser Ile Ala Ala Ala Ala
           675                 680                 685
   Ser Val Val Gly Ala Pro Val Ala Val Val Thr Ser Leu Leu Thr Gly
       690                 695                 700
   Ala Leu Asn Gly Ile Leu Arg Gly Val Gln Gln Pro Ile Ile Glu Lys
   705                 710                 715                 720
   Leu Ala Asn Asp Tyr Ala Arg Lys Ile Asp Glu Leu Gly Gly Pro Gln
                   725                 730                 735
   Ala Tyr Phe Glu Lys Asn Leu Gln Ala Arg His Glu Gln Leu Ala Asn
               740                 745                 750
   Ser Asp Gly Leu Arg Lys Met Leu Ala Asp Leu Gln Ala Gly Trp Asn
           755                 760                 765
   Ala Ser Ser Val Ile Gly Val Gln Thr Thr Glu Ile Ser Lys Ser Ala
       770                 775                 780
   Leu Glu Leu Ala Ala Ile Thr Gly Asn Ala Asp Asn Leu Lys Ser Ala
   785                 790                 795                 800
   Asp Val Phe Val Asp Arg Phe Ile Gln Gly Glu Arg Val Ala Gly Gln
                   805                 810                 815
   Pro Val Val Leu Asp Val Ala Ala Gly Gly Ile Asp Ile Ala Ser Arg
               820                 825                 830
   Lys Gly Glu Arg Pro Ala Leu Thr Phe Ile Thr Pro Leu Ala Ala Pro
           835                 840                 845
   Gly Glu Glu Gln Arg Arg Arg Thr Lys Thr Gly Lys Ser Glu Phe Thr
       850                 855                 860
   Thr Phe Val Glu Ile Val Gly Lys Gln Asp Arg Trp Arg Ile Arg Asp
   865                 870                 875                 880
   Gly Ala Ala Asp Thr Thr Ile Asp Leu Ala Lys Val Val Ser Gln Leu
                   885                 890                 895
   Val Asp Ala Asn Gly Val Leu Lys His Ser Ile Lys Leu Glu Val Ile
               900                 905                 910
   Gly Gly Asp Gly Asp Asp Val Val Leu Ala Asn Ala Ser Arg Ile His
           915                 920                 925
   Tyr Asp Gly Gly Ala Gly Thr Asn Thr Val Ser Tyr Ala Ala Leu Gly
       930                 935                 940
   Arg Gln Asp Ser Ile Thr Val Ser Ala Asp Gly Glu Arg Phe Asn Val
   945                 950                 955                 960
   Arg Lys Gln Leu Asn Asn Ala Asn Val Tyr Arg Glu Gly Val Ala Thr
                   965                 970                 975
   Gln Lys Thr Ala Tyr Gly Lys Arg Thr Glu Asn Val Gln Tyr Arg His
   980 985 990
   Val Glu Leu Ala Arg Val Gly Gln Leu Val Glu Val Asp Thr Leu Glu
   995 1000 1005
   His Val Gln His Ile Ile Gly Gly Ala Gly Asn Asp Ser Ile Thr
   1010 1015 1020
   Gly Asn Ala His Asp Asn Phe Leu Ala Gly Gly Ala Gly Asp Asp
   1025 1030 1035
   Arg Leu Asp Gly Gly Ala Gly Asn Asp Thr Leu Val Gly Gly Glu
   1040 1045 1050
   Gly His Asn Thr Val Val Gly Gly Ala Gly Asp Asp Val Phe Leu
   1055 1060 1065
   Gln Asp Leu Gly Val Trp Ser Asn Gln Leu Asp Gly Gly Ala Gly
   1070 1075 1080
   Val Asp Thr Val Lys Tyr Asn Val His Gln Pro Ser Glu Glu Arg
   1085 1090 1095
   Leu Glu Arg Met Gly Asp Thr Gly Ile His Ala Asp Leu Gln Lys
   1100 1105 1110
   Gly Thr Val Glu Lys Trp Pro Ala Leu Asn Leu Phe Ser Val Asp
   1115 1120 1125
   His Val Lys Asn Ile Glu Asn Leu His Gly Ser Ser Leu Asn Asp
   1130 1135 1140
   Ser Ile Ala Gly Asp Asp Arg Asp Asn Glu Leu Trp Gly Asp Asp
   1145 1150 1155
   Gly Asn Asp Thr Ile His Gly Arg Gly Gly Asp Asp Ile Leu Arg
   1160 1165 1170
   Gly Gly Leu Gly Leu Asp Thr Leu Tyr Gly Glu Asp Gly Asn Asp
   1175 1180 1185
   Ile Phe Leu Gln Asp Asp Glu Thr Val Ser Asp Asp Ile Asp Gly
   1190 1195 1200
   Gly Ala Gly Leu Asp Thr Val Asp Tyr Ser Ala Met Ile His Ala
   1205 1210 1215
   Gly Lys Ile Val Ala Pro His Glu Tyr Gly Phe Gly Ile Glu Ala
   1220 1225 1230
   Asp Leu Ser Glu Gly Trp Val Arg Lys Ala Ala Arg Arg Gly Met
   1235 1240 1245
   Gly Tyr Tyr Asp Ser Val Arg Ser Val Glu Asn Val Ile Gly Thr
   1250 1255 1260
   Ser Met Lys Asp Val Leu Ile Gly Asp Ala Gln Ala Asn Thr Leu
   1265 1270 1275
   Met Gly Gln Gly Gly Asp Asp Thr Val Arg Gly Gly Asp Gly Asp
   1280 1285 1290
   Asp Leu Leu Phe Gly Gly Asp Gly Asn Asp Met Leu Tyr Gly Asp
   1295 1300 1305
   Ala Gly Asn Asp Thr Leu Tyr Gly Gly Leu Gly Asp Asp Thr Leu
   1310 1315 1320
   Glu Gly Gly Ala Gly Asn Asp Trp Phe Gly Gln Thr Pro Ala Arg
   1325 1330 1335
   Glu His Asp Val Leu Arg Gly Gly Ala Gly Val Asp Thr Val Asp
   1340 1345 1350
   Tyr Ser Gln Ala Gly Ala His Ala Gly Val Ala Thr Gly Arg Ile
   1355 1360 1365
   Gly Leu Gly Ile Leu Ala Asp Leu Gly Ala Gly Arg Val Asp Lys
   1370 1375 1380
   Leu Gly Glu Ala Gly Ser Ser Ala Tyr Asp Thr Val Ser Gly Ile
   1385 1390 1395
   Glu Asn Val Val Gly Thr Glu Leu Ala Asp Arg Ile Thr Gly Asp
   1400 1405 1410
   Ala Gln Ala Asn Val Leu Arg Gly Ala Gly Gly Ala Asp Val Leu
   1415 1420 1425
   Ala Gly Gly Glu Gly Asp Asp Val Leu Leu Gly Gly Glu Gly Asp
   1430 1435 1440
   Asp Gln Leu Ser Gly Asp Ala Gly Arg Asp Arg Leu Tyr Gly Glu
   1445 1450 1455
   Ala Gly Asp Asp Trp Phe Phe Gln Asp Ala Ala Asn Ala Gly Asn
   1460 1465 1470
   Leu Leu Asp Gly Gly Asp Gly Asn Asp Thr Val Asp Phe Ser Gly
   1475 1480 1485
   Pro Gly Arg Gly Leu Asp Ala Gly Ala Lys Gly Val Phe Leu Ser
       1490                    1495                    1500
   Leu Gly Lys Gly Phe Ala Ser Leu Met Asp Glu Pro Glu Thr Ser
       1505                    1510                    1515
   Asn Val Leu Arg His Ile Glu Asn Ala Val Gly Ser Val Arg Asp
       1520                    1525                    1530
   Asp Val Leu Ile Gly Asp Ala Gly Ala Asn Val Leu Asn Gly Leu
       1535                    1540                    1545
   Ala Gly Asn Asp Val Leu Ser Gly Gly Ala Gly Asp Asp Val Leu
       1550                    1555                    1560
   Leu Gly Asp Glu Gly Ser Asp Leu Leu Ser Gly Asp Ala Gly Asn
       1565                    1570                    1575
   Asp Asp Leu Phe Gly Gly Gln Gly Asp Asp Thr Tyr Leu Phe Gly
       1580                    1585                    1590
   Ala Gly Tyr Gly His Asp Thr Ile Tyr Glu Ser Gly Gly Gly His
       1595                    1600                    1605
   Asp Thr Ile Arg Ile Asn Ala Gly Ala Asp Gln Leu Trp Phe Ala
       1610                    1615                    1620
   Arg Gln Gly Asn Asp Leu Glu Ile Arg Ile Leu Gly Thr Asp Asp
       1625                    1630                    1635
   Ala Leu Thr Val His Asp Trp Tyr Arg Asp Ala Asp His Arg Val
       1640                    1645                    1650
   Glu Ala Ile His Ala Ala Asn Gln Ala Ile Asp Pro Ala Gly Ile
       1655                    1660                    1665
   Glu Lys Leu Val Glu Ala Met Ala Gln Tyr Pro Asp Pro Gly Ala
       1670                    1675                    1680
   Ala Ala Ala Ala Pro Pro Ala Ala Arg Val Pro Asp Thr Leu Met
       1685                    1690                    1695
   Gln Ser Leu Ala Val Asn Trp Arg
       1700                    1705

<210> 4
<211> 373
<212> PRT
<213> Artificial Sequence

<220>

<223>  AC domain

<400>  4

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Gly Gly Arg Ser
    370

<210>  5
<211>  127
<212>  PRT
<213>  Artificial Sequence

<220>
<223>  Hly linking segment

<400>  5

Lys Ser Ser Pro Asp Val Leu Glu Thr Val Pro Ala Ser Pro Gly Leu
1               5                   10                  15

Arg Arg Pro Ser Leu Gly Ala Val Glu Arg Gln Asp Ser Gly Tyr Asp
            20                  25                  30

Ser Leu Asp Gly Val Gly Ser Arg Ser Phe Ser Leu Gly Glu Val Ser
        35                  40                  45

Asp Met Ala Ala Val Glu Ala Ala Glu Leu Glu Met Thr Arg Gln Val
    50                  55                  60

Leu His Ala Gly Ala Arg Gln Asp Asp Ala Glu Pro Gly Val Ser Gly
65                  70                  75                  80

Ala Ser Ala His Trp Gly Gln Arg Ala Leu Gln Gly Ala Gln Ala Val
                85                  90                  95

Ala Ala Ala Gln Arg Leu Val His Ala Ile Ala Leu Met Thr Gln Phe
            100                 105                 110

Gly Arg Ala Gly Ser Thr Asn Thr Pro Gln Glu Ala Ala Ser Leu
        115                 120                 125

<210>  6
<211>  205
<212>  PRT
<213>  Bordetella bronchiseptica

<400>  6

Met Thr Ile Asp Leu Gly Val Ser Leu Thr Ser Gln Ala Gly Gly Leu
1               5                   10                  15

Gln Gly Ile Asp Leu Lys Ser Met Asp Ile Gln Thr Leu Met Val Tyr
            20                  25                  30

Val Gln Gly Arg Arg Ala Glu Leu Leu Thr Ala Gln Met Gln Thr Gln
        35                  40                  45

Ala Glu Val Val Gln Lys Ala Asn Glu Arg Met Ala Gln Leu Asn Glu
    50                  55                  60

Val Leu Ser Ala Leu Ser Arg Ala Lys Ala Glu Phe Pro Pro Asn Pro
65                  70                  75                  80

Lys Pro Gly Asp Thr Ile Pro Gly Trp Asp Asn Gln Lys Val Ser Arg
                85                  90                  95

Ile Glu Val Pro Leu Asn Asp Ala Leu Arg Ala Ala Gly Leu Thr Gly
            100                 105                 110

Met Phe Glu Ala Arg Asp Gly Gln Val Thr Ala Pro Gly Gly Arg Gly
        115                 120                 125

Thr Gln Val Val Asn Gly Thr Gly Val Met Ala Gly Ser Thr Thr Tyr
    130                 135                 140

Lys Glu Leu Glu Ser Ala Tyr Thr Thr Val Lys Gly Met Leu Asp Thr
145                 150                 155                 160

Ala Ser Asn Thr Gln Gln Met Asp Met Ile Arg Leu Gln Ala Ala Ser
                165                 170                 175

Asn Lys Arg Asn Glu Ala Phe Glu Val Met Thr Asn Thr Glu Lys Arg
            180                 185                 190

Arg Ser Asp Leu Asn Ser Ser Ile Thr Asn Asn Met Arg
        195                 200                 205

<210>  7
<211>  312
<212>  PRT

<213>  Leishmania infantum

<400>  7

Met Asn Tyr Glu Gly His Leu Lys Gly His Arg Gly Trp Val Thr Ser
1               5                   10                  15

Leu Ala Cys Pro Gln Gln Ala Gly Ser Tyr Ile Lys Val Val Ser Thr
            20                  25                  30

Ser Arg Asp Gly Thr Ala Ile Ser Trp Lys Ala Asn Pro Asp Arg His
        35                  40                  45

Ser Val Asp Ser Asp Tyr Gly Leu Pro Ser His Arg Leu Glu Gly His
    50                  55                  60

Thr Gly Phe Val Ser Cys Val Ser Leu Ala His Ala Thr Asp Tyr Ala
65                  70                  75                  80

Leu Thr Ala Ser Trp Asp Arg Ser Ile Arg Met Trp Asp Leu Arg Asn
                85                  90                  95

Gly Gln Cys Gln Arg Lys Phe Leu Lys His Thr Lys Asp Val Leu Ala
            100                 105                 110

Val Ala Phe Ser Pro Asp Asp Arg Leu Ile Val Ser Ala Gly Arg Asp
        115                 120                 125

Asn Val Ile Arg Val Trp Asn Val Ala Gly Glu Cys Met His Glu Phe
    130                 135                 140

Leu Arg Asp Gly His Glu Asp Trp Val Ser Ser Ile Cys Phe Ser Pro
145                 150                 155                 160

Ser Leu Glu His Pro Ile Val Val Ser Gly Ser Trp Asp Asn Thr Ile
                165                 170                 175

Lys Val Trp Asn Val Asn Gly Gly Lys Cys Glu Arg Thr Leu Lys Gly
            180                 185                 190

His Ser Asn Tyr Val Ser Thr Val Thr Val Ser Pro Asp Gly Ser Leu
        195                 200                 205

Cys Ala Ser Gly Gly Lys Asp Gly Ala Ala Leu Leu Trp Asp Leu Ser
    210                 215                 220

Thr Gly Glu Gln Leu Phe Lys Ile Asn Val Glu Ser Pro Ile Asn Gln
225                 230                 235                 240

Ile Ala Phe Ser Pro Asn Arg Phe Trp Met Cys Val Ala Thr Glu Arg
                245                 250                 255

Ser Leu Ser Val Tyr Asp Leu Glu Ser Lys Ala Val Ile Ala Glu Leu
            260                 265                 270

Thr Pro Asp Gly Ala Lys Pro Ser Glu Cys Ile Ser Ile Ala Trp Ser
        275                 280                 285

Ala Asp Gly Asn Thr Leu Tyr Ser Gly His Lys Asp Asn Leu Ile Arg
    290                 295                 300

Val Trp Ser Ile Ser Asp Ala Glu
305                 310

<210> 8
<211> 1335
<212> PRT
<213> Artificial Sequence

<220>
<223> polypeptide

<400> 8

Arg Ser Lys Phe Ser Pro Asp Val Leu Glu Thr Val Pro Ala Ser Pro
1               5                   10                  15

Gly Leu Arg Arg Pro Ser Leu Gly Ala Val Glu Arg Gln Asp Ser Gly
            20                  25                  30

Tyr Asp Ser Leu Asp Gly Val Gly Ser Arg Ser Phe Ser Leu Gly Glu
        35                  40                  45

Val Ser Asp Met Ala Ala Val Glu Ala Ala Glu Leu Glu Met Thr Arg
    50                  55                  60

Gln Val Leu His Ala Gly Ala Arg Gln Asn Asn Ala Gln Pro Gly Val
65                  70                  75                  80

Ser Gly Ala Ser Ala His Trp Gly Gln Arg Ala Leu Gln Gly Ala Gln
                85                  90                  95

Ala Val Ala Ala Ala Gln Arg Leu Val His Ala Ile Ala Leu Met Thr
            100                 105                 110

Gln Phe Gly Arg Ala Gly Ser Thr Asn Thr Pro Gln Glu Ala Ala Ser
        115                 120                 125

Leu Ser Ala Ala Val Phe Gly Leu Gly Glu Ala Ser Ser Ala Val Ala
    130                 135                 140

Glu Thr Val Ser Gly Phe Phe Arg Gly Ser Ser Arg Trp Ala Gly Gly
145                 150                 155                 160

Phe Gly Val Ala Gly Gly Ala Met Ala Leu Gly Gly Gly Ile Ala Ala
                165                 170                 175

Ala Val Gly Ala Gly Met Ser Leu Thr Asp Asp Ala Pro Ala Gly Gln
            180                 185                 190

Lys Ala Ala Ala Gly Ala Glu Ile Ala Leu Gln Leu Thr Gly Gly Thr
        195                 200                 205

Val Glu Leu Ala Ser Ser Ile Ala Leu Ala Leu Ala Ala Arg Gly
    210                 215                 220

Val Thr Ser Gly Leu Gln Val Ala Gly Ala Ser Ala Gly Ala Ala Ala
225                 230                 235                 240

Gly Ala Leu Ala Ala Ala Leu Ser Pro Met Glu Ile Tyr Gly Leu Val
                245                 250                 255

Gln Gln Ser His Tyr Ala Asp Gln Leu Asp Lys Leu Ala Gln Glu Ser
            260                 265                 270

Ser Ala Tyr Gly Tyr Glu Gly Asp Ala Leu Leu Ala Gln Leu Tyr Arg
        275                 280                 285

Asp Lys Thr Ala Ala Glu Gly Ala Val Ala Gly Val Ser Ala Val Leu
    290                 295                 300

Ser Thr Val Gly Ala Ala Val Ser Ile Ala Ala Ala Ser Val Val
305                 310                 315                 320

Gly Ala Pro Val Ala Val Val Thr Ser Leu Leu Thr Gly Ala Leu Asn
                325                 330                 335

Gly Ile Leu Arg Gly Val Gln Gln Pro Ile Ile Glu Lys Leu Ala Asn
            340                 345                 350

Asp Tyr Ala Arg Lys Ile Asp Glu Leu Gly Gly Pro Gln Ala Tyr Phe
        355                 360                 365

Glu Lys Asn Leu Gln Ala Arg His Glu Gln Leu Ala Asn Ser Asp Gly
    370                 375                 380

Leu Arg Lys Met Leu Ala Asp Leu Gln Ala Gly Trp Asn Ala Ser Ser
385                 390                 395                 400

Val Ile Gly Val Gln Thr Thr Glu Ile Ser Lys Ser Ala Leu Glu Leu
                405                 410                 415

Ala Ala Ile Thr Gly Asn Ala Asp Asn Leu Lys Ser Val Asp Val Phe
            420                 425                 430

Val Asp Arg Phe Val Gln Gly Glu Arg Val Ala Gly Gln Pro Val Val
        435                 440                 445

Leu Asp Val Ala Ala Gly Gly Ile Asp Ile Ala Ser Arg Lys Gly Glu
    450                 455                 460

Arg Pro Ala Leu Thr Phe Ile Thr Pro Leu Ala Ala Pro Gly Glu Glu
465                 470                 475                 480

Gln Arg Arg Arg Thr Lys Thr Gly Lys Ser Glu Phe Thr Thr Phe Val
                485                 490                 495

Glu Ile Val Gly Lys Gln Asp Arg Trp Arg Ile Arg Asp Gly Ala Ala
            500                 505                 510

Asp Thr Thr Ile Asp Leu Ala Lys Val Val Ser Gln Leu Val Asp Ala
        515                 520                 525

Asn Gly Val Leu Lys His Ser Ile Lys Leu Asp Val Ile Gly Gly Asp
    530                 535                 540

Gly Asp Asp Val Val Leu Ala Asn Ala Ser Arg Ile His Tyr Asp Gly
545                 550                 555                 560

Gly Ala Gly Thr Asn Thr Val Ser Tyr Ala Ala Leu Gly Arg Gln Asp
                565                 570                 575

Ser Ile Thr Val Ser Ala Asp Gly Glu Arg Phe Asn Val Arg Lys Gln
            580                 585                 590

Leu Asn Asn Ala Asn Val Tyr Arg Glu Gly Val Ala Thr Gln Thr Thr
        595                 600                 605

Ala Tyr Gly Lys Arg Thr Glu Asn Val Gln Tyr Arg His Val Glu Leu
    610                 615                 620

Ala Arg Val Gly Gln Leu Val Glu Val Asp Thr Leu Glu His Val Gln
625                 630                 635                 640

His Ile Ile Gly Gly Ala Gly Asn Asp Ser Ile Thr Gly Asn Ala His
                645                 650                 655

Asp Asn Phe Leu Ala Gly Gly Ser Gly Asp Asp Arg Leu Asp Gly Gly
            660                 665                 670

Ala Gly Asn Asp Thr Leu Val Gly Gly Glu Gly Gln Asn Thr Val Ile
        675                 680                 685

Gly Gly Ala Gly Asp Asp Val Phe Leu Gln Asp Leu Gly Val Trp Ser
    690                 695                 700

Asn Gln Leu Asp Gly Gly Ala Gly Val Asp Thr Val Lys Tyr Asn Val
705                 710                 715                 720

Pge p

His Gln Pro Ser Glu Glu Arg Leu Glu Arg Met Gly Asp Thr Gly Ile
                725                 730                 735

His Ala Asp Leu Gln Lys Gly Thr Val Glu Lys Trp Pro Ala Leu Asn
            740                 745                 750

Leu Phe Ser Val Asp His Val Lys Asn Ile Glu Asn Leu His Gly Ser
        755                 760                 765

Arg Leu Asn Asp Arg Ile Ala Gly Asp Asp Gln Asp Asn Glu Leu Trp
    770                 775                 780

Gly His Asp Gly Asn Asp Thr Ile Arg Gly Arg Gly Gly Asp Asp Ile
785                 790                 795                 800

Leu Arg Gly Gly Leu Gly Leu Asp Thr Leu Tyr Gly Glu Asp Gly Asn
                805                 810                 815

Asp Ile Phe Leu Gln Asp Asp Glu Thr Val Ser Asp Asp Ile Asp Gly
            820                 825                 830

Gly Ala Gly Leu Asp Thr Val Asp Tyr Ser Ala Met Ile His Pro Gly
        835                 840                 845

Arg Ile Val Ala Pro His Glu Tyr Gly Phe Gly Ile Glu Ala Asp Leu
    850                 855                 860

Ser Arg Glu Trp Val Arg Lys Ala Ser Ala Leu Gly Val Asp Tyr Tyr
865                 870                 875                 880

Asp Asn Val Arg Asn Val Glu Asn Val Ile Gly Thr Ser Met Lys Asp
                885                 890                 895

Val Leu Ile Gly Asp Ala Gln Ala Asn Thr Leu Met Gly Gln Gly Gly
            900                 905                 910

Asp Asp Thr Val Arg Gly Gly Asp Gly Asp Asp Leu Leu Phe Gly Gly
        915                 920                 925

Asp Gly Asn Asp Met Leu Tyr Gly Asp Ala Gly Asn Asp Thr Leu Tyr
    930                 935                 940

Gly Gly Leu Gly Asp Asp Thr Leu Glu Gly Gly Ala Gly Asn Asp Trp
945                 950                 955                 960

Phe Gly Gln Thr Gln Ala Arg Glu His Asp Val Leu Arg Gly Gly Asp
                965                 970                 975

Gly Val Asp Thr Val Asp Tyr Ser Gln Thr Gly Ala His Ala Gly Ile
            980                 985                 990

Ala Ala Gly Arg Ile Gly Leu Gly Ile Leu Ala Asp Leu Gly Ala Gly
995 1000 1005

Arg Val Asp Lys Leu Gly Glu Ala Gly Ser Ser Ala Tyr Asp Thr
1010 1015 1020

Val Ser Gly Ile Glu Asn Val Val Gly Thr Glu Leu Ala Asp Arg
1025 1030 1035

Ile Thr Gly Asp Ala Gln Ala Asn Val Leu Arg Gly Ala Gly Gly
1040 1045 1050

Ala Asp Val Leu Ala Gly Gly Glu Gly Asp Asp Val Leu Leu Gly
1055 1060 1065

Gly Asp Gly Asp Asp Gln Leu Ser Gly Asp Ala Gly Arg Asp Arg
1070 1075 1080

Leu Tyr Gly Glu Ala Gly Asp Asp Trp Phe Phe Gln Asp Ala Ala
1085 1090 1095

Asn Ala Gly Asn Leu Leu Asp Gly Gly Asp Gly Arg Asp Thr Val
1100 1105 1110

Asp Phe Ser Gly Pro Gly Arg Gly Leu Asp Ala Gly Ala Lys Gly
1115 1120 1125

Val Phe Leu Ser Leu Gly Lys Gly Phe Ala Ser Leu Met Asp Glu
1130 1135 1140

Pro Glu Thr Ser Asn Val Leu Arg Asn Ile Glu Asn Ala Val Gly
1145 1150 1155

Ser Ala Arg Asp Asp Val Leu Ile Gly Asp Ala Gly Ala Asn Val
1160 1165 1170

Leu Asn Gly Leu Ala Gly Asn Asp Val Leu Ser Gly Gly Ala Gly
1175 1180 1185

Asp Asp Val Leu Leu Gly Asp Glu Gly Ser Asp Leu Leu Ser Gly
1190 1195 1200

Asp Ala Gly Asn Asp Asp Leu Phe Gly Gly Gln Gly Asp Asp Thr
1205 1210 1215

Tyr Leu Phe Gly Val Gly Tyr Gly His Asp Thr Ile Tyr Glu Ser
1220 1225 1230

Gly Gly Gly His Asp Thr Ile Arg Ile Asn Ala Gly Ala Asp Gln
1235 1240 1245

Leu Trp Phe Ala Arg Gln Gly Asn Asp Leu Glu Ile Arg Ile Leu
    1250                1255                1260

Gly Thr Asp Asp Ala Leu Thr Val His Asp Trp Tyr Arg Asp Ala
    1265                1270                1275

Asp His Arg Val Glu Ile Ile His Ala Ala Asn Gln Ala Val Asp
    1280                1285                1290

Gln Ala Gly Ile Glu Lys Leu Val Glu Ala Met Ala Gln Tyr Pro
    1295                1300                1305

Asp Pro Gly Ala Ala Ala Ala Ala Pro Pro Ala Ala Arg Val Pro
    1310                1315                1320

Asp Thr Leu Met Gln Ser Leu Ala Val Asn Trp Arg
    1325                1330                1335

<210>  9
<211>  1590
<212>  PRT
<213>  Artificial Sequence

<220>
<223>  CyaA Bordetella B - Bsp22 antigen

<400>  9

Met Gly Thr Val Asn Gly Leu Glu Gln Leu Glu Ser Ile Ile Asn Phe
1               5                   10                  15

Glu Lys Leu Thr Glu Trp Thr Ser Ser Asn Val Met Glu Glu Arg Lys
            20                  25                  30

Ile Lys Val Tyr Leu Pro Arg Ala Arg Ala Ala Thr Ser Thr Ile Asp
        35                  40                  45

Leu Gly Val Ser Leu Thr Ser Gln Ala Gly Gly Leu Gln Gly Ile Asp
    50                  55                  60

Leu Lys Ser Met Asp Ile Gln Thr Leu Met Val Tyr Val Gln Gly Arg
65                  70                  75                  80

Arg Ala Glu Leu Leu Thr Ala Gln Met Gln Thr Gln Ala Glu Val Val
                85                  90                  95

Gln Lys Ala Asn Glu Arg Met Ala Gln Leu Asn Glu Val Leu Ser Ala
            100                 105                 110

Leu Ser Arg Ala Lys Ala Glu Phe Pro Pro Asn Pro Lys Pro Gly Asp
        115                 120                 125

Thr Ile Pro Gly Trp Asp Asn Gln Lys Val Ser Arg Ile Glu Val Pro
    130                 135                 140

Leu Asn Asp Ala Leu Arg Ala Ala Gly Leu Thr Gly Met Phe Glu Ala
145                 150                 155                 160

Arg Asp Gly Gln Val Thr Ala Pro Gly Gly Arg Gly Thr Gln Val Val
                165                 170                 175

Asn Gly Thr Gly Val Met Ala Gly Ser Thr Thr Tyr Lys Glu Leu Glu
            180                 185                 190

Ser Ala Tyr Thr Thr Val Lys Gly Met Leu Asp Thr Ala Ser Asn Thr
        195                 200                 205

Gln Gln Met Asp Met Ile Arg Leu Gln Ala Ala Ser Asn Lys Arg Asn
    210                 215                 220

Glu Ala Phe Glu Val Met Thr Asn Thr Glu Lys Arg Arg Ser Asp Leu
225                 230                 235                 240

Asn Ser Ser Ile Thr Asn Asn Met Arg Thr Gly Ala Ala Pro Ser Arg
                245                 250                 255

Ser Lys Phe Ser Pro Asp Val Leu Glu Thr Val Pro Ala Ser Pro Gly
            260                 265                 270

Leu Arg Arg Pro Ser Leu Gly Ala Val Glu Arg Gln Asp Ser Gly Tyr
        275                 280                 285

Asp Ser Leu Asp Gly Val Gly Ser Arg Ser Phe Ser Leu Gly Glu Val
    290                 295                 300

Ser Asp Met Ala Ala Val Glu Ala Ala Glu Leu Glu Met Thr Arg Gln
305                 310                 315                 320

Val Leu His Ala Gly Ala Arg Gln Asp Asp Ala Glu Pro Gly Val Ser
                325                 330                 335

Gly Ala Ser Ala His Trp Gly Gln Arg Ala Leu Gln Gly Ala Gln Ala
            340                 345                 350

Val Ala Ala Ala Gln Arg Leu Val His Ala Ile Ala Leu Met Thr Gln
        355                 360                 365

Phe Gly Arg Ala Gly Ser Thr Asn Thr Pro Gln Glu Ala Ala Ser Leu
    370                 375                 380

Ser Ala Ala Val Phe Gly Leu Gly Glu Ala Ser Ser Ala Val Ala Glu
385                 390                 395                 400

Thr Val Ser Gly Phe Phe Arg Gly Ser Ser Arg Trp Ala Gly Gly Phe
                405                 410                 415

Gly Val Ala Gly Gly Ala Met Ala Leu Gly Gly Gly Ile Ala Ala Ala
            420                 425                 430

Val Gly Ala Gly Met Ser Leu Thr Asp Asp Ala Pro Ala Gly Gln Lys
        435                 440                 445

Ala Ala Ala Gly Ala Glu Ile Ala Leu Gln Leu Thr Gly Gly Thr Val
    450                 455                 460

Glu Leu Ala Ser Ser Ile Ala Leu Ala Leu Ala Ala Ala Arg Gly Val
465                 470                 475                 480

Thr Ser Gly Leu Gln Val Ala Gly Ala Ser Ala Gly Ala Ala Ala Gly
                485                 490                 495

Ala Leu Ala Ala Ala Leu Ser Pro Met Glu Ile Tyr Gly Leu Val Gln
            500                 505                 510

Gln Ser His Tyr Ala Asp Gln Leu Asp Lys Leu Ala Gln Glu Ser Ser
        515                 520                 525

Ala Tyr Gly Tyr Glu Gly Asp Ala Leu Leu Ala Gln Leu Tyr Arg Asp
    530                 535                 540

Lys Thr Ala Ala Glu Gly Ala Val Ala Gly Val Ser Ala Val Leu Ser
545                 550                 555                 560

Thr Val Gly Ala Ala Val Ser Ile Ala Ala Ala Ala Ser Val Val Gly
                565                 570                 575

Ala Pro Val Ala Val Val Thr Ser Leu Leu Thr Gly Ala Leu Asn Gly
            580                 585                 590

Ile Leu Arg Gly Val Gln Gln Pro Ile Ile Glu Lys Leu Ala Asn Asp
        595                 600                 605

Tyr Ala Arg Lys Ile Asp Glu Leu Gly Gly Pro Gln Ala Tyr Phe Glu
    610                 615                 620

Lys Asn Leu Gln Ala Arg His Glu Gln Leu Ala Asn Ser Asp Gly Leu
625                 630                 635                 640

Arg Lys Met Leu Ala Asp Leu Gln Ala Gly Trp Asn Ala Ser Ser Val
                645                 650                 655

Ile Gly Val Gln Thr Thr Glu Ile Ser Lys Ser Ala Leu Glu Leu Ala
            660                 665                 670

Ala Ile Thr Gly Asn Ala Asp Asn Leu Lys Ser Val Asp Val Phe Val
        675                 680                 685

Asp Arg Phe Val Gln Gly Glu Arg Val Ala Gly Gln Pro Val Val Leu
    690                 695                 700

Asp Val Ala Ala Gly Gly Ile Asp Ile Ala Ser Arg Lys Gly Glu Arg
705                 710                 715                 720

Pro Ala Leu Thr Phe Ile Thr Pro Leu Ala Ala Pro Gly Glu Glu Gln
                725                 730                 735

Arg Arg Arg Thr Lys Thr Gly Lys Ser Glu Phe Thr Thr Phe Val Glu
            740                 745                 750

Ile Val Gly Lys Gln Asp Arg Trp Arg Ile Arg Asp Gly Ala Ala Asp
        755                 760                 765

Thr Thr Ile Asp Leu Ala Lys Val Val Ser Gln Leu Val Asp Ala Asn
    770                 775                 780

Gly Val Leu Lys His Ser Ile Lys Leu Asp Val Ile Gly Gly Asp Gly
785                 790                 795                 800

Asp Asp Val Val Leu Ala Asn Ala Ser Arg Ile His Tyr Asp Gly Gly
                805                 810                 815

Ala Gly Thr Asn Thr Val Ser Tyr Ala Ala Leu Gly Arg Gln Asp Ser
            820                 825                 830

Ile Thr Val Ser Ala Asp Gly Glu Arg Phe Asn Val Arg Lys Gln Leu
        835                 840                 845

Asn Asn Ala Asn Val Tyr Arg Glu Gly Val Ala Thr Gln Thr Thr Ala
    850                 855                 860

Tyr Gly Lys Arg Thr Glu Asn Val Gln Tyr Arg His Val Glu Leu Ala
865                 870                 875                 880

Arg Val Gly Gln Val Val Glu Val Asp Thr Leu Glu His Val Gln His
                885                 890                 895

Ile Ile Gly Gly Ala Gly Asn Asp Ser Ile Thr Gly Asn Ala His Asp
            900                 905                 910

Asn Phe Leu Ala Gly Gly Ser Gly Asp Asp Arg Leu Asp Gly Gly Ala
        915                 920                 925

Gly Asn Asp Thr Leu Val Gly Gly Glu Gly Gln Asn Thr Val Ile Gly
    930                 935                 940

Gly Ala Gly Asp Asp Val Phe Leu Gln Asp Leu Gly Val Trp Ser Asn
945                 950                 955                 960

Gln Leu Asp Gly Gly Ala Gly Val Asp Thr Val Lys Tyr Asn Val His
                965                 970                 975

Gln Pro Ser Glu Glu Arg Leu Glu Arg Met Gly Asp Thr Gly Ile His
            980                 985                 990

Ala Asp Leu Gln Lys Gly Thr Val  Glu Lys Trp Pro Ala  Leu Asn Leu
        995                 1000                 1005

Phe Ser  Val Asp His Val Lys  Asn Ile Glu Asn Leu  His Gly Ser
    1010                 1015                 1020

Arg Leu  Asn Asp Arg Ile Ala  Gly Asp Asp Gln Asp  Asn Glu Leu
    1025                 1030                 1035

Trp Gly  His Asp Gly Asn Asp  Thr Ile Arg Gly Arg  Gly Gly Asp
    1040                 1045                 1050

Asp Ile  Leu Arg Gly Gly Leu  Gly Leu Asp Thr Leu  Tyr Gly Glu
    1055                 1060                 1065

Asp Gly  Asn Asp Ile Phe Leu  Gln Asp Asp Glu Thr  Val Ser Asp
    1070                 1075                 1080

Asp Ile  Asp Gly Lys Ala Gly  Leu Asp Thr Val Asp  Tyr Ser Ala
    1085                 1090                 1095

Met Ile  His Pro Gly Arg Ile  Val Ala Pro His Glu  Tyr Gly Phe
    1100                 1105                 1110

Gly Ile  Glu Ala Asp Leu Ser  Arg Glu Trp Val Arg  Lys Ala Ser
    1115                 1120                 1125

Ala Leu  Gly Val Asp Tyr Tyr  Asp Asn Val Arg Asn  Val Glu Asn
    1130                 1135                 1140

Val Ile  Gly Thr Ser Met Lys  Asp Val Leu Ile Gly  Asp Ala Gln
    1145                 1150                 1155

Ala Asn  Thr Leu Met Gly Gln  Gly Gly Asp Asp Thr  Val Arg Gly
    1160                 1165                 1170

Gly Asp  Gly Asp Asp Leu Leu  Phe Gly Gly Asp Gly  Asn Asp Met
    1175                 1180                 1185

Leu Tyr  Gly Asp Ala Gly Asn  Asp Thr Leu Tyr Gly  Gly Leu Gly
    1190                 1195                 1200

Asp Asp Thr Leu Glu Gly Gly Ala Gly Asn Asp Trp Phe Gly Gln
    1205                1210                1215

Thr Gln Ala Arg Glu His Asp Val Leu Arg Gly Gly Asp Gly Val
    1220                1225                1230

Asp Thr Val Asp Tyr Ser Gln Thr Gly Ala His Ala Gly Ile Ala
    1235                1240                1245

Ala Gly Arg Ile Gly Leu Gly Ile Leu Ala Asp Leu Gly Ala Gly
    1250                1255                1260

Arg Val Asp Lys Leu Gly Glu Ala Gly Ser Ser Ala Tyr Asp Thr
    1265                1270                1275

Val Ser Gly Ile Glu Asn Val Val Gly Thr Glu Leu Ala Asp Arg
    1280                1285                1290

Ile Thr Gly Asp Ala Gln Ala Asn Val Leu Arg Gly Ala Gly Gly
    1295                1300                1305

Ala Asp Val Leu Ala Gly Gly Glu Gly Asp Asp Val Leu Leu Gly
    1310                1315                1320

Gly Asp Gly Asp Asp Gln Leu Ser Gly Asp Ala Gly Arg Asp Arg
    1325                1330                1335

Leu Tyr Gly Glu Ala Gly Asp Asp Trp Phe Phe Gln Asp Ala Ala
    1340                1345                1350

Asn Ala Gly Asn Leu Leu Asp Gly Gly Asp Gly Arg Asp Thr Val
    1355                1360                1365

Asp Phe Ser Gly Pro Gly Arg Gly Leu Asp Ala Gly Ala Lys Gly
    1370                1375                1380

Val Phe Leu Ser Leu Gly Lys Gly Phe Ala Ser Leu Met Asp Glu
    1385                1390                1395

Pro Glu Thr Ser Asn Val Leu Arg Asn Ile Glu Asn Ala Val Gly
    1400                1405                1410

Ser Ala Arg Asp Asp Val Leu Ile Gly Asp Ala Gly Ala Asn Val
    1415                1420                1425

Leu Asn Gly Leu Ala Gly Asn Asp Val Leu Ser Gly Gly Ala Gly
    1430                1435                1440

Asp Asp Val Leu Leu Gly Asp Glu Gly Ser Asp Leu Leu Ser Gly
    1445                1450                1455

Asp Ala Gly Asn Asp Asp Leu Phe Gly Gly Gln Gly Asp Asp Thr
    1460                1465                1470

Tyr Leu Phe Gly Val Gly Tyr Gly His Asp Thr Ile Tyr Glu Ser
    1475                1480                1485

Gly Gly Gly His Asp Thr Ile Arg Ile Asn Ala Gly Ala Asp Gln
    1490                1495                1500

Leu Trp Phe Ala Arg Gln Gly Asn Asp Leu Glu Ile Arg Ile Leu
    1505                1510                1515

Gly Thr Asp Asp Ala Leu Thr Val His Asp Trp Tyr Arg Asp Ala
    1520                1525                1530

Asp His Arg Val Glu Ile Ile His Ala Ala Asn Gln Ala Val Asp
    1535                1540                1545

Gln Ala Gly Ile Glu Lys Leu Val Glu Ala Met Ala Gln Tyr Pro
    1550                1555                1560

Asp Pro Gly Ala Ala Ala Ala Ala Pro Pro Ala Ala Arg Val Pro
    1565                1570                1575

Asp Thr Leu Met Gln Ser Leu Ala Val Asn Trp Arg
    1580                1585                1590

<210> 10
<211> 1698
<212> PRT
<213> Artificial Sequence

<220>
<223> Chimeric protein CyaA-P36

<400> 10

Met Gly Thr Val Asn Gly Leu Glu Gln Leu Glu Ser Ile Ile Asn Phe
1               5                   10                  15

Glu Lys Leu Thr Glu Trp Thr Ser Ser Asn Val Met Glu Glu Arg Lys
            20                  25                  30

Ile Lys Val Tyr Leu Pro Arg Ala Arg Ala Ala Thr Ser Met Asn Tyr
        35                  40                  45

Glu Gly His Leu Lys Gly His Arg Gly Trp Val Thr Ser Leu Ala Cys
    50                  55                  60

Pro Gln Gln Ala Gly Ser Tyr Ile Lys Val Val Ser Thr Ser Arg Asp
65                  70                  75                  80

Gly Thr Ala Ile Ser Trp Lys Ala Asn Pro Asp Arg His Ser Val Asp
                85                  90                  95

Ser Asp Tyr Gly Leu Pro Ser His Arg Leu Glu Gly His Thr Gly Phe
            100                 105                 110

Val Ser Cys Val Ser Leu Ala His Ala Thr Asp Tyr Ala Leu Thr Ala
        115                 120                 125

Ser Trp Asp Arg Ser Ile Arg Met Trp Asp Leu Arg Asn Gly Gln Cys
    130                 135                 140

Gln Arg Lys Phe Leu Lys His Thr Lys Asp Val Leu Ala Val Ala Phe
145                 150                 155                 160

Ser Pro Asp Asp Arg Leu Ile Val Ser Ala Gly Arg Asp Asn Val Ile
                165                 170                 175

Arg Val Trp Asn Val Ala Gly Glu Cys Met His Glu Phe Leu Arg Asp
            180                 185                 190

Gly His Glu Asp Trp Val Ser Ser Ile Cys Phe Ser Pro Ser Leu Glu
        195                 200                 205

His Pro Ile Val Val Ser Gly Ser Trp Asp Asn Thr Ile Lys Val Trp
    210                 215                 220

Asn Val Asn Gly Gly Lys Cys Glu Arg Thr Leu Lys Gly His Ser Asn
225                 230                 235                 240

Tyr Val Ser Thr Val Thr Val Ser Pro Asp Gly Ser Leu Cys Ala Ser
                245                 250                 255

Gly Gly Lys Asp Gly Ala Ala Leu Leu Trp Asp Leu Ser Thr Gly Glu
            260                 265                 270

Gln Leu Phe Lys Ile Asn Val Glu Ser Pro Ile Asn Gln Ile Ala Phe
        275                 280                 285

Ser Pro Asn Arg Phe Trp Met Cys Val Ala Thr Glu Arg Ser Leu Ser
    290                 295                 300

Val Tyr Asp Leu Glu Ser Lys Ala Val Ile Ala Glu Leu Thr Pro Asp
305                 310                 315                 320

Gly Ala Lys Pro Ser Glu Cys Ile Ser Ile Ala Trp Ser Ala Asp Gly
                325                 330                 335

Asn Thr Leu Tyr Ser Gly His Lys Asp Asn Leu Ile Arg Val Trp Ser
            340                 345                 350

Ile Ser Asp Ala Glu Thr Gly Ala Ala Pro Ser Arg Ser Lys Phe Ser
        355                 360                 365

Pro Asp Val Leu Glu Thr Val Pro Ala Ser Pro Gly Leu Arg Arg Pro
    370                 375                 380

Ser Leu Gly Ala Val Glu Arg Gln Asp Ser Gly Tyr Asp Ser Leu Asp
385                 390                 395                 400

Gly Val Gly Ser Arg Ser Phe Ser Leu Gly Glu Val Ser Asp Met Ala
                405                 410                 415

Ala Val Glu Ala Ala Glu Leu Glu Met Thr Arg Gln Val Leu His Ala
            420                 425                 430

Gly Ala Arg Gln Asp Asp Ala Glu Pro Gly Val Ser Gly Ala Ser Ala
        435                 440                 445

His Trp Gly Gln Arg Ala Leu Gln Gly Ala Gln Ala Val Ala Ala Ala
    450                 455                 460

Gln Arg Leu Val His Ala Ile Ala Leu Met Thr Gln Phe Gly Arg Ala
465                 470                 475                 480

Gly Ser Thr Asn Thr Pro Gln Glu Ala Ala Ser Leu Ser Ala Ala Val
                485                 490                 495

Phe Gly Leu Gly Glu Ala Ser Ser Ala Val Ala Glu Thr Val Ser Gly
            500                 505                 510

Phe Phe Arg Gly Ser Ser Arg Trp Ala Gly Gly Phe Gly Val Ala Gly
        515                 520                 525

Gly Ala Met Ala Leu Gly Gly Gly Ile Ala Ala Ala Val Gly Ala Gly
    530                 535                 540

Met Ser Leu Thr Asp Asp Ala Pro Ala Gly Gln Lys Ala Ala Ala Gly
545                 550                 555                 560

Ala Glu Ile Ala Leu Gln Leu Thr Gly Gly Thr Val Glu Leu Ala Ser
                565                 570                 575

Ser Ile Ala Leu Ala Leu Ala Ala Ala Arg Gly Val Thr Ser Gly Leu
            580                 585                 590

Gln Val Ala Gly Ala Ser Ala Gly Ala Ala Ala Gly Ala Leu Ala Ala
        595                 600                 605

Ala Leu Ser Pro Met Glu Ile Tyr Gly Leu Val Gln Gln Ser His Tyr
    610                 615                 620

Ala Asp Gln Leu Asp Lys Leu Ala Gln Glu Ser Ser Ala Tyr Gly Tyr
625                 630                 635                 640

Glu Gly Asp Ala Leu Leu Ala Gln Leu Tyr Arg Asp Lys Thr Ala Ala
                645                 650                 655

Glu Gly Ala Val Ala Gly Val Ser Ala Val Leu Ser Thr Val Gly Ala
            660                 665                 670

Ala Val Ser Ile Ala Ala Ala Ala Ser Val Val Gly Ala Pro Val Ala
        675                 680                 685

Val Val Thr Ser Leu Leu Thr Gly Ala Leu Asn Gly Ile Leu Arg Gly
    690                 695                 700

Val Gln Gln Pro Ile Ile Glu Lys Leu Ala Asn Asp Tyr Ala Arg Lys
705                 710                 715                 720

Ile Asp Glu Leu Gly Gly Pro Gln Ala Tyr Phe Glu Lys Asn Leu Gln
                725                 730                 735

Ala Arg His Glu Gln Leu Ala Asn Ser Asp Gly Leu Arg Lys Met Leu
            740                 745                 750

Ala Asp Leu Gln Ala Gly Trp Asn Ala Ser Ser Val Ile Gly Val Gln
        755                 760                 765

Thr Thr Glu Ile Ser Lys Ser Ala Leu Glu Leu Ala Ala Ile Thr Gly
    770                 775                 780

Asn Ala Asp Asn Leu Lys Ser Val Asp Val Phe Val Asp Arg Phe Val
785                 790                 795                 800

Gln Gly Glu Arg Val Ala Gly Gln Pro Val Val Leu Asp Val Ala Ala
                805                 810                 815

Gly Gly Ile Asp Ile Ala Ser Arg Lys Gly Glu Arg Pro Ala Leu Thr
            820                 825                 830

Phe Ile Thr Pro Leu Ala Ala Pro Gly Glu Glu Gln Arg Arg Arg Thr
        835                 840                 845

Lys Thr Gly Lys Ser Glu Phe Thr Thr Phe Val Glu Ile Val Gly Lys
    850                 855                 860

Gln Asp Arg Trp Arg Ile Arg Asp Gly Ala Ala Asp Thr Thr Ile Asp
865                 870                 875                 880

Leu Ala Lys Val Val Ser Gln Leu Val Asp Ala Asn Gly Val Leu Lys
                885                 890                 895

His Ser Ile Lys Leu Asp Val Ile Gly Gly Asp Gly Asp Asp Val Val
            900                 905                 910

Leu Ala Asn Ala Ser Arg Ile His Tyr Asp Gly Gly Ala Gly Thr Asn
        915                 920                 925

Thr Val Ser Tyr Ala Ala Leu Gly Arg Gln Asp Ser Ile Thr Val Ser
    930                 935                 940

Ala Asp Gly Glu Arg Phe Asn Val Arg Lys Gln Leu Asn Asn Ala Asn
945                 950                 955                 960

Val Tyr Arg Glu Gly Val Ala Thr Gln Thr Thr Ala Tyr Gly Lys Arg
                965                 970                 975

Thr Glu Asn Val Gln Tyr Arg His Val Glu Leu Ala Arg Val Gly Gln
            980                 985                 990

Val Val Glu Val Asp Thr Leu Glu His Val Gln His Ile Ile Gly Gly
        995                 1000                1005

Ala Gly Asn Asp Ser Ile Thr Gly Asn Ala His Asp Asn Phe Leu
    1010                1015                1020

Ala Gly Gly Ser Gly Asp Asp Arg Leu Asp Gly Gly Ala Gly Asn
    1025                1030                1035

Asp Thr Leu Val Gly Gly Glu Gly Gln Asn Thr Val Ile Gly Gly
    1040                1045                1050

Ala Gly Asp Asp Val Phe Leu Gln Asp Leu Gly Val Trp Ser Asn
    1055                1060                1065

Gln Leu Asp Gly Gly Ala Gly Val Asp Thr Val Lys Tyr Asn Val
    1070                1075                1080

His Gln Pro Ser Glu Glu Arg Leu Glu Arg Met Gly Asp Thr Gly
    1085                1090                1095

Ile His Ala Asp Leu Gln Lys Gly Thr Val Glu Lys Trp Pro Ala
    1100                1105                1110

Leu Asn Leu Phe Ser Val Asp His Val Lys Asn Ile Glu Asn Leu
    1115                1120                1125

His Gly Ser Arg Leu Asn Asp Arg Ile Ala Gly Asp Asp Gln Asp
    1130                1135                1140

Asn Glu Leu Trp Gly His Asp Gly Asn Asp Thr Ile Arg Gly Arg
    1145                1150                1155

Gly Gly Asp Asp Ile Leu Arg Gly Gly Leu Gly Leu Asp Thr Leu
    1160                1165                1170

Tyr Gly Glu Asp Gly Asn Asp Ile Phe Leu Gln Asp Asp Glu Thr
    1175                1180                1185

Val Ser Asp Asp Ile Asp Gly Gly Ala Gly Leu Asp Thr Val Asp
    1190                1195                1200

Tyr Ser Ala Met Ile His Pro Gly Arg Ile Val Ala Pro His Glu
    1205                1210                1215

Tyr Gly Phe Gly Ile Glu Ala Asp Leu Ser Arg Glu Trp Val Arg
    1220                1225                1230

Lys Ala Ser Ala Leu Gly Val Asp Tyr Tyr Asp Asn Val Arg Asn
    1235                1240                1245

Val Glu Asn Val Ile Gly Thr Ser Met Lys Asp Val Leu Ile Gly
    1250                1255                1260

Asp Ala Gln Ala Asn Thr Leu Met Gly Gln Gly Gly Asp Asp Thr
    1265                1270                1275

Val Arg Gly Gly Asp Gly Asp Asp Leu Leu Phe Gly Gly Asp Gly
    1280                1285                1290

Asn Asp Met Leu Tyr Gly Asp Ala Gly Asn Asp Thr Leu Tyr Gly
    1295                1300                1305

Gly Leu Gly Asp Asp Thr Leu Glu Gly Gly Ala Gly Asn Asp Trp
    1310                1315                1320

Phe Gly Gln Thr Gln Ala Arg Glu His Asp Val Leu Arg Gly Gly
    1325                1330                1335

Asp Gly Val Asp Thr Val Asp Tyr Ser Gln Thr Gly Ala His Ala
    1340                1345                1350

Gly Ile Ala Ala Gly Arg Ile Gly Leu Gly Ile Leu Ala Asp Leu
    1355                1360                1365

Gly Ala Gly Arg Val Asp Lys Leu Gly Glu Ala Gly Ser Ser Ala
    1370                1375                1380

Tyr Asp Thr Val Ser Gly Ile Glu Asn Val Val Gly Thr Glu Leu
    1385                1390                1395

Ala Asp Arg Ile Thr Gly Asp Ala Gln Ala Asn Val Leu Arg Gly
    1400                1405                1410

Ala Gly Gly Ala Asp Val Leu Ala Gly Gly Glu Gly Asp Asp Val
    1415                1420                1425

Leu Leu Gly Gly Asp Gly Asp Asp Gln Leu Ser Gly Asp Ala Gly
    1430                1435                1440

Arg Asp Arg Leu Tyr Gly Glu Ala Gly Asp Asp Trp Phe Phe Gln
    1445                1450                1455

Asp Ala Ala Asn Ala Gly Asn Leu Leu Asp Gly Gly Asp Gly Arg
    1460                1465                1470

Asp Thr Val Asp Phe Ser Gly Pro Gly Arg Gly Leu Asp Ala Gly
    1475                1480                1485

Ala Lys Gly Val Phe Leu Ser Leu Gly Lys Gly Phe Ala Ser Leu
    1490                1495                1500

Met Asp Glu Pro Glu Thr Ser Asn Val Leu Arg Asn Ile Glu Asn
    1505                1510                1515

Ala Val Gly Ser Ala Arg Asp Asp Val Leu Ile Gly Asp Ala Gly
    1520                1525                1530

Ala Asn Val Leu Asn Gly Leu Ala Gly Asn Asp Val Leu Ser Gly
    1535                1540                1545

Gly Ala Gly Asp Asp Val Leu Leu Gly Asp Glu Gly Ser Asp Leu
    1550                1555                1560

Leu Ser Gly Asp Ala Gly Asn Asp Asp Leu Phe Gly Gly Gln Gly
    1565                1570                1575

Asp Asp Thr Tyr Leu Phe Gly Val Gly Tyr Gly His Asp Thr Ile
    1580                1585                1590

Tyr Glu Ser Gly Gly Gly His Asp Thr Ile Arg Ile Asn Ala Gly
    1595                1600                1605

Ala Asp Gln Leu Trp Phe Ala Arg Gln Gly Asn Asp Leu Glu Ile
    1610                1615                1620

Arg Ile Leu Gly Thr Asp Asp Ala Leu Thr Val His Asp Trp Tyr
    1625                1630                1635

Arg Asp Ala Asp His Arg Val Glu Ile Ile His Ala Ala Asn Gln
    1640                1645                1650

Ala Val Asp Gln Ala Gly Ile Glu Lys Leu Val Glu Ala Met Ala
    1655                1660                1665

Gln Tyr Pro Asp Pro Gly Ala Ala Ala Ala Ala Pro Pro Ala Ala
    1670                1675                1680

Arg Val Pro Asp Thr Leu Met Gln Ser Leu Ala Val Asn Trp Arg
    1685                1690                1695